Europäisches Patentamt

European Patent Office

Office européen des brevets

(19)

(11) **EP 1 498 825 A1**

(12) **EUROPEAN PATENT APPLICATION**
published in accordance with Art. 158(3) EPC

(43) Date of publication:
**19.01.2005 Bulletin 2005/03**

(21) Application number: **03715637.9**

(22) Date of filing: **31.03.2003**

(51) Int Cl.⁷: **G06F 17/18**, G06F 17/30,
C12N 15/00, C12Q 1/68,
G01N 33/574

(86) International application number:
**PCT/JP2003/004059**

(87) International publication number:
**WO 2003/085548 (16.10.2003 Gazette 2003/42)**

(84) Designated Contracting States:
**AT BE BG CH CY CZ DE DK EE ES FI FR GB GR
HU IE IT LI LU MC NL PT RO SE SI SK TR**
Designated Extension States:
**AL LT LV MK**

(30) Priority: **04.04.2002 JP 2002102743
04.12.2002 JP 2002352645**

(71) Applicant: **ISHIHARA SANGYO KAISHA, LTD.
Osaka-shi, Osaka 550-0002 (JP)**

(72) Inventors:
• **ISHIKAWA, T.,Chuo Kenkyusho,ISHIHARA
SANGYO KAISHA
Kusatsu-shi, Shiga 525-0025 (JP)**
• **KUME, T.,Chuo Kenkyusho,ISHIHARA SANGYO
KAISHA
Kusatsu-shi, Shiga 525-0025 (JP)**

(74) Representative: **HOFFMANN - EITLE
Patent- und Rechtsanwälte
Arabellastrasse 4
81925 München (DE)**

(54) **APPARATUS AND METHOD FOR ANALYZING DATA**

(57)    In a data analysis system for determining a correlation model between biological conditions and expression levels of a plurality of genes and/or quantities of intracellular substances, A data set is constructed by using the biological conditions or changes of the biological conditions probabilistically generated with time as object variables, and the expression levels of the plural genes and/or the quantities of intracellular substances as explanatory variables. The explanatory variables contained in the data are selected, and cross validation is calculated for a correlation model containing the selected explanatory variables and object variables to assess the results. Selection of the explanatory variables, calculation of cross validation and discriminative assessment of the result is repeated until no further improvement of the cross validation is observed to determine a partial least square model. This permits an effective processing method to provide multivariable gene information.

*Fig. 2*

EP 1 498 825 A1

**EP 1 498 825 A1**

**Description**

TECHNICAL FIELD

The invention relates to multivariate analysis between a biological condition and gene expression levels and/or quantities of intracellular substances, and a measuring instrument therefor and a testing method based on the analysis.

BACKGROUND ART

Since the declaration of complete decoding of human genome in June 2000, it is said that a post-genome era has begun for elucidating how genetic information written in the genome is expressed and functions. In the progress of human genome program methodologies for measuring the state of genome expression have been developed. An oligonucleotide array and a microchip have been developed for measuring transcriptome (mRNA). Recently, mass spectroscopy has also been advanced in addition to conventional two-dimensional electrophoresis as a measuring method of proteomes (proteins). Other advanced technologies such as antibody chip have also attracted attention. These measuring technologies are epoch-making over conventional technologies because they can collectively obtain biological state parameters in a short period of time.

Technologies for efficiently measuring the gene expression are as follows. For measuring transcriptome (a total assembly of mRNAs), a DNA chip has been developed, in which a plurality of DNAs are retained on a substrate in order to detect mRNAs complementary to the DNAs. Examples of the representative DNA chips include a gene chip and a DNA micro-array. Two-dimensional electrophoresis, an antibody chip and mass spectroscopy have been used for identifying the proteome (an assembly of proteins). Measurements of metabolome (an assembly of metabolites including metabolic intermediates) have also been attempted by using mass spectrometry, and this method is now in progress.

Since the conditions of the cells in a body can be described precisely by the expression of gene products, more accurate diagnosis may be expected by these measurements even when conventional diagnosis markers give insufficient information. Examples of such studies are as follows.

P. O. Brown et al. measured the transcriptome in the cells of lymphoma patients (DLBCL) with a DNA chip, and classified the patients into different clusters of benign and malignant lymphoma by cluster analysis (Nature 403 (3), 503-11 (2000)). However, since this method cannot obtain a model of causal relation (correlation), it cannot decide which gene is important to what extent.

A. Alaiya et al. measured proteomes in the cells of 44 uterine cancer patients by two-dimensional electrophoresis, and constructed a diagnosis model based on a partial least square method from data of 22 patients in order to elucidate malignancy of the disease (Int. J. Cancer, 86, 731-36 (2000); Electrophoresis, 21, 1210-17 (2000); International Publication WO 00/70340). The results of cross-validation is improved ($Q^2 = 0.84$) by selecting 170 variables having large loading from 1,553 variables in a full model, and correct answers were obtained in a ratio of 11/18 with respect to degree of seriousness (three stages) of the remaining 18 patients. The authors indicated that the cross-validation method can be used as an index for constructing a model. However, a full model should be established in this method before loading is obtained. Further, alternative methods for selecting the variables have not yet been devised.

J. Khan et al. measured the cells of infant cancer patients with DNA chips, and elucidated malignancy of the disease by means of an artificial neural network (Nature Medicine, 7(6), 673-79 (2001)). The authors measured transcriptomes (6,567 genes) of 88 infant cancer patients (SRBCT), the data from 63 patients were compressed into 10 dimensions by principal component analysis, and then an artificial neural network diagnosis model is constructed. Effective high-ranking genes were selected by cross-validation method, and the best result (100%) is obtained with respect to 96 genes. The remaining 25 patients were predicted by this model to obtain a result of 93 to 100%. However, a full model should also be established in this method before obtaining effective genes, and alternative methods for obtaining variables have not yet been devised. While this method is valid in the case having variables as small as 10 dimensions, it cannot be applied to a case of a large number of variables.

Recently, D. M. Rocke and D. V. Nguyen reported the use of the partial lest-square method for the analysis of the DNA chip (International Publication WO 02/25405; Bioinformatics 18(1), 39-50 (2002); Bioinformatics 18(9), 1216-26 (2002); Bioinformatics 18(12), 1625-32 (2002)). It is reported that good result is obtained by using latent variables of the partial least square method as explanatory variables of multivariate analysis such as linear discriminant analysis. This method is usable because the partial least square method is able to simultaneously perform reduction of dimension and model fitting. Reported examples show that the partial least square method is excellent as a model construction method for DNA chip information. However, the report does not mention applicability of the partial least-square method as means for selecting the expression level of important genes, and the method also involves the problems in the study by A. Alaiya et al. because the data are analyzed by using all explanatory variables selected in advance.

More accurate (higher resolution) diagnosis may be expected by using gene expression information even when sufficient information cannot be obtained with conventional markers. A result of a measurement of gene expression has a feature that a large amount of information is obtained, and the data cannot be used fully unless the data are effectively processed since the large amount of information is included. Accordingly, effective information processing is inevitable for acquiring useful knowledge. While cluster analysis is used mainly today as described above, other methods such as principal component analysis have also been employed. However, since cluster analysis and principal component analysis do not use supervised learning, a model of causal relationships (correlation) of disease conditions cannot be obtained. In other words, it is a weak point that the results of analysis do not tell which gene is important to what extent. While the partial least square method is a powerful multivariate analysis method for simultaneously performing reduction of dimension and model fitting, we often encounter a situation that significant results cannot be obtained when the number of the variables is very large. Accordingly, an effective information processing is desired for acquiring useful knowledge from a large amount of information of gene expression. Also expected are efficient measuring instruments and test methods based on the results of information processing.

DISCLOSURE OF THE INVENTION

(Technical Problems to be Solved by the Invention)

An object of the invention is to provide an effective information processing method for information on multivariate gene expression, information on intracellular substances and the like.

Another object of the invention is to provide an effective examination method therefor.

(Means for Solving the Problems)

A data analysis system according to the invention determines a correlation model with a biological condition or a change in the biological condition probabilistically generated with time as an object variable and gene expression levels and/or quantities of intracellular substances as explanatory variables. The system has an input means for receiving a sample set including data on the biological condition or data from which the biological condition is derived, or data on the change in the biological condition probabilistically generated with time, and the expression levels of a plurality of genes and/or the quantities of intr acellular substances; (A) a selection means for selecting explanatory variables; (B) a calculation means for calculating a result of cross validation by executing a partial least square method, or for calculating a result of cross validation by executing a partial least square method by using a conversion result as the object variable, the conversion result being obtained by converting a probability that no change occurs by applying a life table according to Kaplan-Meier method or Cutler-Ederer method to the data on the change in the biological condition, based on or without an assumed distribution; (C) a discriminative assessing means for judging adoption and non-adoption of the explanatory variables by assessing the result of the calculation by the calculation means; and (D) a decision means for deciding a partial least square model by continuously improving a function having the result of the cross validation of the partial least square model as at least one of independent variables by using the selection means (A), the calculation means (B) and the judging means (C). For example, the selection means sequentially selects the explanatory variables, or selects them by using a genetic algorithm. The calculation means calculates a result of the cross validation by, for example, applying a partial least square method. after excluding one or more samples, sequentially. The judging means determines, for example, a representative value of an error between the object variable on the biological condition predicted by the gene expression of the excluded sample in each calculation and the object variable on the biological condition of the excluded sample. The result of the cross validation is judged to be improved if the representative value of the error is reduced. The discriminative assessment of the cross validation is repeated while the explanatory variables are selected by inclusion or exclusion. Alternatively, it may be used as a criterion of discriminative assessment, instead of using the cross validation whether a function having the result of cross validation of the partial least square model as at least one of independent variables is improved or not. The decision means determines the partial least square model by, for example, continuously improving the result of cross validation of the partial least square model by repeatedly using the selection means, calculation means and judging means. The selection means and calculation means may be executed with a plurality of computers. When a correlation model is constructed optimally, the explanatory variables are selected based on the result of cross validation, and a good model is obtained by reducing the dimensions of the explanatory variables.

The conversion based on an assumed distribution or without the assumed distribution is performed in order to analyze the probability of the change in the biological condition with a polynomial of the explanatory variables. The conversion based on the assumed distribution include a division of a logarithm of the probability with a negative sign added after the logarithmic conversion by the observation time of the change in the condition, a division of a logarithmic value of a negative value after a logarithmic conversion of the probability by the observation time of the change of the conditions, or a division of a calculated value of probit (probability unit) conversion of a value obtained by subtracting the probability from 1 by the observation time of the change of the conditions. On the other hand, a logit transformation may be used when the conversion is performed without assumed distribution. A conversion method may be appropriately selected by deciding what kind of assumption is valid for the distribution or not. An example of the function having the result of cross validation of the partial least square model as at least one of independent variables include a function of the representative value of the error and the number of the selected explanatory variables, or the function may include other independent variables. The function is desirably a monotonically decreasing function of the representative value of the error, or a monotonically decreasing function of the number of the explanatory variables. The function can desirably be calculated simply in order to prevent increase in the amount of calculation, and an example thereof is a function -PRESS $\times$ alpha$^{NP}$ wherein PRESS is a predicted residual error sum of squares, NP is the number of the explanatory variables used, and alpha is a real number larger than 1. Other functions available include -PRESS $\times$ (NP + beta)$^{gamma}$ and -PRESS $\times$ (beta - NP)$^{-gamma}$ where gamma denotes a positive real number.

A usual statistical method or multivariate analysis method may be applied when the number of the explanatory variables is reduced. A better model can be obtained in the invention by using the explanatory variables selected by using the partial least square method as the explanatory variables in a statistical method or multivariate analysis method. Otherwise, a better model may be obtained by using latent variables in the partial least square method with the selected explanatory variables as the explanatory variables in the statistical method or multivariate analysis method. The latent variables are used usually in the partial least square method. Compression of dimensions in the partial least square method model fitting is performed by extracting latent variables ($T_{ik}$) of small dimensions common as a background of the object variables ($Y_{il}$) and the explanatory variables ($X_{ij}$).

$$Y_{il} = \sum Q_{kl} \times T_{ik} + F_{il,}$$

and

$$X_{ij} = \sum P_{kj} \times T_{ik} + E_{ij,}$$

wherein i denotes the sample number, 1 denotes the number of the object variable, j denotes the number of the explanatory variables, k denotes the number of the latent variable, and F and E denote residuals.

Examples of the statistical method and multivariate analysis method include a multiple regression analysis, linear discriminant analysis, adaptive least square method, logistic linear regression, proportional hazards analysis, discriminant analysis with Mahalanobis distance, kNN method and artificial neural network.

The inventors also find that the explanatory variables can be arbitrarily selected by optimizing a function having the number of the explanatory variables as a second independent variable, besides the result of cross validation such as $Q^2$, PRESS values. When the desirable range of the number NP of the extracted explanatory variables is determined in relation to the number of the samples in the usual statistical method and multivariate analysis method, the function may arbitrarily be modified according to the desired number of selection to be attained. When the function is expressed as -PRESS $\times$ alpha$^{NP}$, for example, the constant alpha is desirably in the range of 1.0 to 3.0, more desirably in the range of 1.0 to 2.0, in order to decrease the number of the explanatory variables in the range of several to several tens variables. The selection of the variables may have the same advantage in other functions f(PRESS, NP) such as f(PRESS_MP/alpha, MP + 1) $\approx$ f(PRESS_MP, MP) around the number MP of the actually selected explanatory variables and the value of PRESS_MP with respect to the selection of the variables. By using an appropriate function as described above, the number of NP of the explanatory variables to be selected can be within a desirable range. A model obtained by the statistical method or multivariate analysis method can be constructed by further selecting the explanatory variables employed in the model determined by using the result of the cross validation. Consequently, data analysis is possible by using the statistical method or multivariate analysis method whose nature has been fully elucidated.

A correlation model between the change in the biological condition probabilistically generated with time and the gene expression levels and/or the quantities of the intracellular substances can be determined by using a quantity derived from the biological condition generated with time as the object variable. "The change in the biological condition probabilistically generated with time" is, for example, a survival time. The partial least square method is combined with Kaplan-Meier method or Cutler-Ederer method and logit transformation. The object variable in the partial least square method is obtained by calculating a probability that no change occurs by applying a life table according to Kaplan-Meier method or Cutler-Ederer method to the data on the change in the biological condition generated with time and by performing logit transformation of the probability. Logit values are calculated with an equation, $\text{logit} = \log\{P/(1-P)\}$, based on a ratio (probability) $P$ of a class in the classified data. The result of cross validation is calculated with the partial least square method by using the logit value as the object variable. The survival time is analyzed after extracting the explanatory variables by taking the cross validation with the partial least square method into consideration as described above.

A usual statistical method or multivariate analysis method becomes applicable when the number of the explanatory variables is reduced. Then, a model of a statistical method or multivariable method for elucidating the change in the biological condition probabilistically generated with time is constructed by using the explanatory variables or latent variables thereof employed in the determined model. For example, a better model may be obtained by applying another statistical method or multivariate analysis method (e.g., a proportional hazard method or a linear regression analysis applied to a parametric distribution) by using the explanatory variables determined with the logit value as the object variable. The proportional hazard method developed by Cox takes the time into consideration in the analysis of the survival ratio, and it can deal with multivariables. The analysis in the proportional hazard method uses a hazard value that influences the survival ratio defined for each individual of observation and a function which derives the hazard value. Kaplan-Meier method shows the survival ratio for the total set or for each group thereof. The parametric distribution means a probability distribution calculated from a normal distribution proposed by Gauss, and an exponential distribution, a Weibull analysis or a logarithmic normal distribution is used in the analysis of the survival time. In the fitting to the exponential or other distributions, a polynomial is included in the formula, and the explanatory variables is selected by taking the result of the cross validation of the partial least square method into consideration.

The expression levels of the genes and/or the quantities of the intracellular substances received as the explanatory variables by the input means are not restricted to the measured absolute concentrations of the substances. Instead, they may be calculated and processed values, relative values or quantities indirectly representing the quantities of the substances. For example, a correlation model that can directly correlate the object variable representing the biological condition with mass spectra may be constructed by taking an advantage of the ability of the mass spectra for measuring protein expressions. A single spot in a DNA chip (gene chip) manufactured by Affymetrix Co. does not always identify an expression of a single gene, and an expression of a single gene may be identified only when a plurality of spots are used. Accordingly, a correlation model for directly elucidating the biological condition may be obtained by using the measured values of respective spots as the explanatory variables. Moreover, each peak in the electrophoresis pattern of a protein may not be assigned to a single protein, and a plurality of proteins may be overlapped in a single peak. Accordingly, each peak intensity may be used as an explanatory variable for elucidating a biological condition. This is evident from the fact that Alaiya et al. employed the peak intensities of the electrophoresis pattern as the explanatory variables of uterine cancer. As mentioned above, an experimental approach starting from the substances in a living body (cells) totally has attracted attention in the research fields such as transcriptome analysis, proteome analysis and metabolome analysis in the post-sequence era. Absolute quantification of respective substances is not essential, and measured values directly or indirectly expressing the quantities of substances quantified by such an experimental method and values obtained by processing them may serve as the explanatory variables for elucidating the biological condition. Further, a more effective result of analysis may often be obtained by adding other explanatory variables such as clinical interview data other than the explanatory variables for elucidating the quantities of the substances as described above.

A data analysis method according to the invention determines a correlation model with a biological condition or a change in the biological condition probabilistically generated with time as an object variable and gene expression levels and/or quantities of intracellular substances as explanatory variables. The method has the steps of: receiving a sample set including data on the biological condition or data from which the biological condition is derived, or data on the change in the biological condition probabilistically generated with time, and the expression levels of a plurality of genes and/or the quantities of intracellular substances; (A) selecting the explanatory variables; (B) calculating a result of cross validation by executing a partial least square method, or calculating a result of cross validation by executing a partial least square method by using a conversion result as the object variable, the conversion result being obtained by converting a probability that no change occurs when a life table is applied according to Kaplan-Meier method or Cutler-Ederer method to the data on the change in the biological condition, based on or without an assumed distribution; (C) judging adoption and non-adoption of the explanatory variables by assessing the result of the calculation by the calculation in the calculating step (B); and (D) deciding a partial least square model by continuously improving a function having the result of the cross validation of the partial least square model as at least one of independent variables by using the selecting step (A), the calculating step (B) and the judging step (C).

In the data analysis method, in the selection step, the explanatory variables are sequentially selected or they are selected by using a genetic algorithm. In the calculation step, the result of cross validation is calculated, for example, with the partial least square method by excluding one or more samples, sequentially. In the discriminative assessment step, a representative value of an error between the object variable on the biological condition predicted from gene expressions in the excluded samples and the object variable indicating the biological conditions of the excluded samples in each calculation is determined in each calculation based on the result obtained in the calculation step. The result of the cross validation is judged to be improved when the representative value of the error is reduced. The discriminative assessment of the cross validation is repeated while the explanatory variables are selected. In the decision step, a partial least square model is determined, for example, by continuously improving the result of the cross validation of the partial least square model by repeatedly executing the selection step, calculation step and discriminative assessment step. The selection step and calculation step may be executed by using a plurality of computers.

A data analysis program executable with a computer according to the invention determines a correlation model by using biological condition or change in the biological condition probabilistically generated with time as object variables and the expression levels of a plurality of genes and/or the quantities of intracellular substances as explanatory variables. The program has the steps of: receiving a sample set including data on the biological condition or data from which the biological condition is derived, or data on the change in the biological condition probabilistically generated with time, and the expression levels of a plurality of genes and/or the quantities of intracellular substances; (A) selecting the explanatory variables; (B) calculating a result of cross validation by executing a partial least square method, or calculating a result of cross validation by executing a partial least square method by using a conversion result as the object variable, the conversion result being obtained by converting a probability that no change occurs when a life table is applied according to Kaplan-Meier method or Cutler-Ederer method to the data on the change in the biological condition, based on or without an assumed distribution; (C) judging adoption and non-adoption of the explanatory variables by assessing the result of the calculation by the calculation in the calculating step (B); and (D) deciding a partial least square model by continuously improving a function having the result of the cross validation of the partial least square model as at least one of independent variables by using the selecting step (A), the calculating step (B) and the judging step (C).

In the data analysis program, in the selection step, the explanatory variables are sequentially selected or they are selected by using a genetic algorithm. In the calculation step, the result of cross validation is calculated, for example, with the partial least square method by excluding one or more samples, sequentially. In the discriminative assessment step, a representative value of an error between the object variable on the biological condition predicted from gene expressions in the excluded samples and the object variable indicating the biological conditions of the excluded samples in each calculation is determined in each calculation based on the result obtained in the calculation step. The result of the cross validation is judged to be improved when the representative value of the error of a monotonously decreasing function having the representative value as an independent variable is reduced. The discriminative assessment of the cross validation is repeated while the explanatory variables are selected. In the decision step, a partial least square model is determined, for example, by continuously improving the result of the cross validation of the partial least square model by repeatedly executing the selection step, calculation step and discriminative assessment step. The selection step and calculation step may be executed by using a plurality of computers. The explanatory variables are not included at all or the full explanatory variables are included in the initial state in the selection of the explanatory variables.

In the data analysis program, the biological condition includes a measured value representing the type of disease, a measured value representing degree of critical of disease, a result of diagnosis representing the types of disease, a result of diagnosis representing degree of critical of disease, or a value obtained by secondary processing thereof. For example, predicting the survival time of a patient gives important information for deciding a therapeutic program including QOL (quality of life) and a life design as will be shown below in examples, so that the program can provide a valuable diagnosis model for the society. Predicting possibility of recurrence of cancer may provide valuable information for making a therapeutic program considering QOL and for deciding selection of therapy by a doctor or a patient.

A data analysis apparatus according to the invention includes an input means for receiving a determined correlation model and explanatory variables adopted in the model for the samples to be predicted, and a means for predicting and discriminating the biological condition of the samples based on the received explanatory variables. A data analysis method according to the invention includes the steps of receiving the determined correlation model and the explanatory variables adopted in the model for the samples to be predicted, and predicting and discriminating the biological condition of the samples based on the received explanatory variables. A data analysis program according to the invention has the steps of receiving the determined correlation model and the explanatory variables adopted in the model for the samples to be predicted, and predicting and discriminating the biological condition of the samples based on the received explanatory variables.

The computer-readable recording medium according to the invention records any one of the programs described above.

In an intracellular substance measuring device, a detection method thereof, or a method for examining degree of critical of diffuse large cell lymphoma based on the detection according to the invention, the device detects expression of at least one gene selected substantially in a gene group of gene bank accession numbers U15085, M23452, X52479, U70426, H57330 and S69790. Preferably, expression of at least one gene selected substantially from a gene group of gene bank accession numbers U03398, M65066, AK001546, BC003536, X00437, U12979, H96306, AA830781 and AA804793 may further be detected.

In an intracellular substance measuring device, a measuring method thereof, or a method for examining degree of critical of breast cancer based on the detection according to the invention, intracellular substances containing gene products comprising substantially genes of gene bank accession numbers AA598572, AA703058 and AA453345 are detected. Preferably, intracellular substances containing substantially at least one gene product selected from a gene group of gene bank accession numbers AA406242, H73335, W84753, N71160, AA054669, N32820 and R05667 are further detected.

In an intracellular substance measuring device, a measuring method thereof, and a method for examining possibility of recurrence of breast cancer based on the detection according to the invention, intracellular substances containing gene products comprising substantially genes of gene bank accession numbers W84753, H08581, AA045730 and AI250654 are detected. Preferably, intracellular substances containing substantially at least one gene product of genes selected from a gene group consisting of gene bank accession numbers AA448641, R78516, R05934, AA629838 and H53037 are further detected.

In an intracellular substance measuring device, a measuring method thereof, and a method for examining possibility of recurrence of breast cancer based on the detection, intracellular substances containing gene products substantially comprising genes of gene bank accession numbers AA434397, T83209, N53427, N29639, AA485739, AA425861, H84871, T64312, T59518 and AA037488 are detected. Preferably, intracellular substances containing gene products substantially of a gene of gene bank accession number AA406231 is detected further.

In an intracellular substance measuring device, a measuring method thereof, and a method for examining possibility of recurrence of breast cancer based on the detection according to the invention, intracellular substances containing gene products substantially of genes of gene bank accession numbers H 11482, T64312 and AA045340 are detected.

Examples of the intracellular substance measuring device include a DNA chip such as a DNA micro-array, a gene chip, an oligo-DNA chip, an electrochemical DNA chip (an ECA chip), a fibrous DNA chip, a magnetic bead DNA chip (a PSS) and a bobbin DNA chip (PSS), a macro-array, an antibody chip and a kit of measuring reagents. A measuring instrument may be integrated with these devices.

BRIEF DESCRIPTION OF THE DRAWINGS

Fig. 1 is a block diagram of a system for analyzing gene expression.
Fig. 2 is a flowchart of an analysis software program.
Fig. 3 is a flowchart for calculating a result CV of cross validation.
Fig. 4 is a first flowchart for constructing a model for selecting variables.
Fig. 5 is a second flowchart for constructing a model for selecting variables.
Fig. 6 is a third flowchart for constructing a model for selecting variables.
Fig. 7 is a fourth flowchart for constructing a model for selecting variables.
Fig. 8 is a fifth flowchart for constructing a model for selecting variables.
Fig. 9 is a graph of the result of the least square model.
Fig. 10 shows comparisons between the survival time of patients and diagnostic indices of DLBCL of patients.
Fig. 11 shows a plot of the survival time of the DLBCL of patients in Example 2 against diagnosis score.
Fig. 12 shows a plot of the survival time of the breast cancer patients in Example 3 against diagnosis score.
Fig. 13 is a plot of the survival time against diagnosis score when $P \geq 0.0005$ is employed as a variable deletion criterion of the breast cancer patients in Example 3.
Fig. 14 shows a plot of the recurrence time against diagnosis score of the breast cancer patients in Example 7.
Fig. 15 is a plot of the recurrence time against diagnosis score when $P \geq 0.025$ is employed as a variable deletion criterion of the breast cancer patients in Example 7.
Fig. 16 shows optimization of the partial least square model by using the genetic algorithm in Example 9.
Fig. 17 shows four topologies in hierarchical artificial neural networks in Example 10.
Fig. 18 is a graph of a relationship between the survival time and diagnosis score of breast cancer patients in the proportional hazards model with the latent variables in Example 11.
Fig. 19 is a graph of a relationship between the survival time and predicted values of the diagnosis score of breast cancer patients in the proportional hazards model with the latent variables in Example 11.

BEST MODE FOR CARRYING OUT THE INVENTION

Embodiments of the invention will be described hereinafter with reference to the appended drawings.

Determination of a correlation model between a selected biological condition and the gene expression levels and/or the amount of the intracellular substances will be described below. The term "gene expression" is used in the invention to include expression of mRNA (transcriptome) and proteins (proteome) formed as a result of transcription of mRNAs. The amount of the intracellular substances means, for example, a metabolome corresponding to all the metabolic products including intermediate metabolites. For example, each sample data in the analysis of the transcriptome (mRNAs) and proteome (proteins) has a biological condition and gene expression levels. Each sample contains a large amount of gene expression levels of not smaller than 1,000 genes. While the biological conditions are, for example, disease types and diagnostic indices of diseases, they may include biological information in a more general sense. The "diagnostic index of disease" includes the extent of progress as well as the type, degree of critical and degree of seriousness of the disease. Since the measured data such as the gene expression levels have a large amount of information, an effective multivariate analysis using a computer is required.

In data acquisition, a biological condition such as a diagnostic index is discriminated for a number of samples, cellular fluids are sampled from the samples, and the gene expression levels and the like of many gene products in the cellular fluids are measured. A correlation model such as a partial least square model is obtained in the data analysis according to an embodiment of the invention by inputting levels of the gene expression level and the like and a biological condition such as a diagnostic index. A causal analysis is performed by using a multivariate analysis program in a computer by assuming the diagnostic index as the object variable and the gene expression levels and/or the quantities of intracellular substances as the explanatory variables in order to obtain information on the importance and effectiveness of respective explanatory variables. The object variable is not always required to be a measured value itself. A value obtained by logit transformation or a discrete value representing a group may be used in order to obtain a more significant result of data analysis.

The inventors find in the field of medical diagnosis with gene expression that a good correlation model (e.g., a partial least square model) can be obtained by selecting variables so as to optimize a function having the result of cross-validation used in the data analysis as at least one of independent variables. In the cross-validation, the data at hand are divided into a plurality of groups, and a model is obtained by fitting for only a part of the divided data groups (a training set) while predictive power of the model is examined by predicting a remaining data group (a test set). The cross-validation has been used for selection of dimensions of the latent variables in the conventional partial least square method (PLS). However, in the partial least square method of the invention the latent dimension is fixed to one, and a function having the cross validation (e.g., a predictable error of a sum of squares) as at least one of the independent variables is optimized while one or more input variables (explanatory variables) are selected successively. However, the advantage of the invention may be obtained without restricting the dimension of the latent dimension to one. Consequently, it is found that a good and powerfully predictable correlation model can be obtained even when a significant correlation model cannot be obtained by using the full variables. A stable correlation model may be obtained by the sequential selection of variables with the cross-validation. The inventors also find that a good correlation model based on statistics and multivariate analysis other than the partial least square method can be obtained by selecting the explanatory variables by appropriately determining a function form, and that a correlation model suitable for an object variable for describing a respective biological condition can be obtained. "Optimization" as used herein means that the cross validation is improved until no further improvement is possible in the corresponding analysis conditions when the explanatory variables are selected, and it never means that the cross validation can find an optimum combination among the entire combinations of the explanatory variables. By using the variable selection, a small number of factors for determining the diseases can be identified, and an inexpensive diagnostic device such as a DNA chip, an antibody chip and a DNA content vector can be designed, so that the variable selection itself is valuable. This variable selection can be employed together with a various type of variable selection conditions to be set in advance.

As mentioned above, the explanatory variables are sequentially selected based on the cross validation. A function having the cross validation as at least one independent variable is used for the selection. When an explanatory variable is added temporarily but the function is judged not to be improved by adding the explanatory variable, the addition of the explanatory variable is canceled. However, the addition of the explanatory variable is accepted when judged that the function is improved. Alternatively, when an explanatory variable is excluded temporarily but the function is judged not to be improved by excluding the explanatory variable, the exclusion of the explanatory variable is canceled, while the exclusion of the explanatory variable is accepted when judged to be improved. When one or more explanatory variables are selected, assessment of the cross validation is performed as follows. Partial least square models are obtained by excluding one or more samples among the n samples sequentially, and in each model the object variable for the biological condition of the excluded sample(s) predictable from the gene expression levels of the excluded sample(s) and then a representative value of errors relative to the object variable is determined. The "representative value" as used herein refers to a value characterizing the data such as a sum, an average, a maximum, a median and a mode value. The cross validation is judged to be improved when the value of the function having the representative value of the error as at least one of the independent variables is reduced, so that the corresponding explanatory variable is added or excluded. The function is continuously improved by sequentially assessing the cross validation while the explanatory variable(s) are sequentially selected. The selection of the explanatory variables is completed when no improvement is judged to be observed because the cross validation have been optimized. Consequently, an optimum partial least square model having a limited number of the selected explanatory variables is obtained. Actually, the processing described above is executable by employing a predicted residual error sum of squares (PRESS) as the index of the value of the cross validation obtained with a calculation device, and by determining to adopt an explanatory variable when the value of the predicted residual error sum of squares is reduced at a rate equal to or smaller than a threshold for the explanatory variable.

A processing for avoiding overfitting should be devised in an analysis method for the causal relationship. "Overfitting" as used herein means that a model has no predictable ability except for the data used for the model fitting since the model fails to describe a true correlation due to a too large number of the explanatory variables although prediction of the model happen to agree with the data used for the model fitting. The partial least square method is used as the correlation model here, and it is said relatively powerful against the problem of overfitting because it is a powerful multivariate analysis method that can perform both reduction of dimension and model fitting at the same time. However, one is confronted with a situation that a significant result cannot be obtained when a large number of variables are dealt as in the analysis of gene expression. Since the methods used by Alaiya and Khan described in the background art are valid on the premise that a full variables model is significantly valid, they cannot be used generally for selecting the variables. On the contrary, the invention can reduce overfitting by selecting the variables so as to optimize the result of cross validation prediction. In addition, the invention provides a method not needing a preprocessing such as principal component analysis. On the contrary, in the prior art methods, since a significant model cannot be obtained when the number of the explanatory variables is quite large, the dimension is compressed with a preprocessing such as the principal component analysis based on the full explanatory variables, and the explanatory variables obtained by the preprocessing are used for analysis. However, in this method, the full explanatory variables used as the basis of the construction of the model are inevitable for prediction. For example, all the genes used for constructing the model are necessary to be held on a diagnostic gene chip, or the variables should be selected, with a different method. On the contrary, in the invention, since the number of the explanatory variables is decreased by selecting them, only the genes corresponding to the selected explanatory variables are required to be held on a diagnostic gene chip as far as they correspond to the gene expression level.

Todeschini et al. obtained a multiple regression analysis model by selecting the variables so as to optimize the cross validation by a genetic algorithm in order to predict decomposition of organic compounds in the atmosphere (P. Gramatics, V. Consonni & R. Todeschini, Chemosphere 38(5), 1371-78 (1999)). The model is constructed by using 53 compounds and 175 descriptors ($Q^2 = 0.79$) to result in selection of seven variables and prediction of 98 compounds ($Q^2 = 0.75$). This method is the same as the method in the invention in that the variables are selected so as to optimize the cross validation. However, because a multiple regression model is used, the number of the variables selected among the explanatory variables is forced to be small, and the method cannot be applied to the analysis of the gene expression levels and/or the quantities of intracellular substances. According to the inventors' experience, in a method for optimizing $Q^2$ or PRESS, the number of the selected explanatory variables ranges from hundreds to several hundreds, and multiple regression model cannot be applied to the analysis. Todeschini et al. does not mention any effective method for selecting the explanatory variables. This is because the number of the candidate of the original explanatory variables is 175 at most, and it is not necessary to select the explanatory variables. However, in the analytical field of gene expression quite different from the method described above, the number of the candidates of the explanatory variables amounts to several hundred and several thousands, even several tens thousands, for several tens to several hundreds of samples. Accordingly, a quite different idea from prior art is required.

When a correlation model between a biological condition and gene expression levels an/or the quantities of the intracellular substances is determined, a good correlation model is obtained in this embodiment by selecting the explanatory variables by sequentially adding or excluding an explanatory variable so as to optimize a function having the cross validation as at least one independent variable. Advantages of this approach are as follows, as will be apparent from the examples explained below.

A) Important genes and mechanisms working in the background of a disease and a biological phenomenon can be estimated or identified and they can be understood well.
B) An inexpensive diagnostic material (such as a DNA chip and an antibody chip) can be designed by selecting a target to crucial gene products and intracellular substances.

In this embodiment the explanatory variables are selected sequentially so as to optimize a function having the cross validation as at least one independent variable. For example, following means may be combined: means for selecting the explanatory variables as represented by a stepwise method, means for calculating the cross validation by applying the partial least square method as represented by the leave-one-out method, means for discriminatively assessing adoption and non-adoption of a explanatory variable by assessing the result in the calculation means. In other word, the adoption and non-adoption of a selected explanatory variable is decided by selecting at least one explanatory variable among m explanatory variables, calculating cross validation by executing the partial least square method, and assessing the calculation result. In the assessment, based on the result of the calculation, a representative value of the error is obtained in each calculation between the object variable on the biological conditions predicted from gene expression of the sample excluded and the object variable on the biological condition of the excluded sample. Then, the selection of the explanatory variable is decided when the representative value of the error of the monotonically decreasing function, having the representative value of the error as at least one of independent variables, decreases. The function having the cross validation of the partial least square method as at least one of independent variables is continuously improved by using the selection, calculation and discriminative assessment as described above until no improvement is observed, and the partial least square model is determined. The sample is excluded one after one sequentially in this embodiment (leave-one-out method). However, a plurality of samples may be excluded to assess the cross validation (leave-n-out method), or another method such as three-fold method used by Khan et al. may be used. In the three-fold method, the explanatory variables are randomly shuffled to divide them into three groups. A model is constructed using two of the three groups, and it is assessed for the remaining one group. Alternatively, a stepwise method or a non-linear algorithm such as a genetic algorithm may be used for selecting the explanatory variables. The range of the search may be restricted when some conditions are known with respect to the selection of the variables.

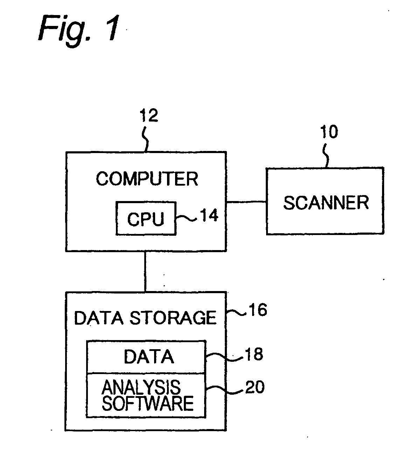

Next, data acquisition and analysis will be described below in detail. Fig. 1 shows a gene expression analysis system. Diagnostic indices (e.g., type of diseases and degree of disease advancement) of some samples are discriminated in advance for data acquisition, and cell fluids are collected from the sample to measure the expression levels of many gene products using DNA chips. A cofocal laser scanner 10 (e.g., a 428 array scanner manufactured by Affymetrix Co.) is used for the measurement of mRNA by absorbance. The measured data are sent to a computer 12 and analyzed there. The computer 12 has a usual structure having a CPU 14, and measured data 18 and a data analysis program 20 are stored in a recording medium (e.g., a hard disk) in a data storage unit (e.g., a hard disk drive) 16 connected to the computer. The data 18 is analyzed with the data analysis program 20 to determine a correlation model between the biological condition and the gene expression levels.

Selection of the explanatory variables and calculation for applying the partial least square method to the prediction of cross-validation may be executed by a plurality of computers 12 in order to accelerate the calculation by dispersing the calculation of prediction of cross-validation to the computers.

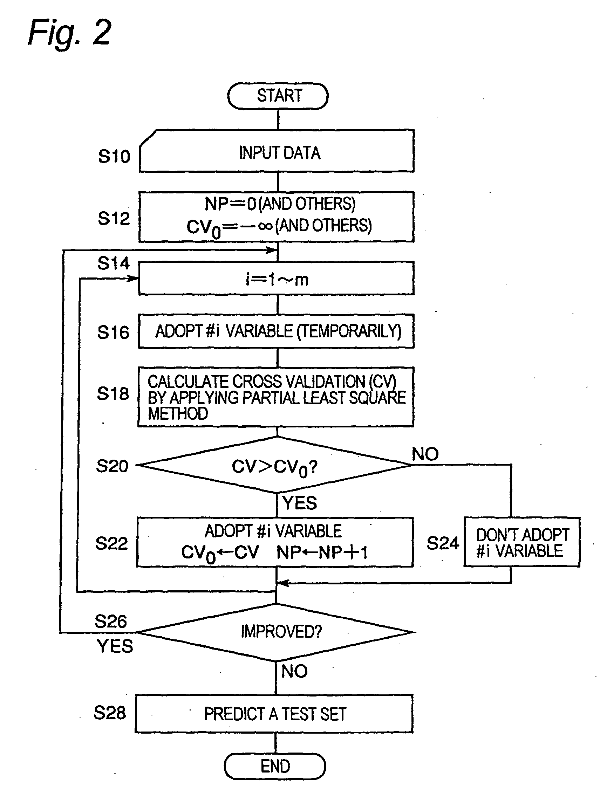

Fig. 2 shows a flowchart of the data analysis program 20 executed by the computer 12 for obtaining a correlation model between the biological condition and the gene expression levels. While -PRESS is employed as a function having the cross validation of the partial least square model as at least one of independent variables, this does not restrict the invention, and different functions are used in Examples 2 to 5, in fact. In a first step, data for preparing the correlation model are received (S10). The data has been collected with a DNA chip. The input data (a sample set) has a respective object variable (e.g., diagnostic index) and m (e.g., 2,000) explanatory variables (e.g., the gene expression levels). Otherwise, the data of the test set are received in addition to the above data (a training set). The test set as used herein does not mean a data group for assessment of cross-validation, but it may be a data group for testing predictive power after the model is determined.

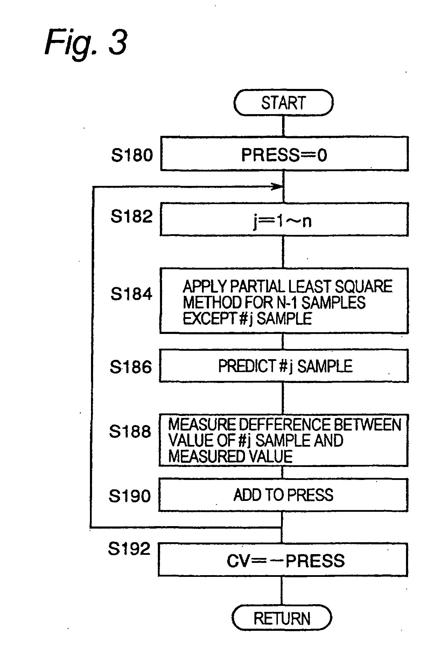

The number of the explanatory variables selected at the initial setting is set to zero, and the best value $CV_0$ of the cross validation CV is set to $-\infty$ (S12). Then, the explanatory variables are selected. The number i indexing an explanatory variable is set to 1 (S14), the i-th variable (gene expression level) is temporarily adopted (S16), and the partial least square method is applied to calculate the cross validation (S18, see Fig. 3). Leave-one-out processing is employed in the calculation. For example, in this processing, a result predicted in the training set having 50 samples, by remaining 49 samples after excluding sequentially one sample from all the first to 50th samples is compared with a result predicted from each of the excluded samples, and the temporarily selected explanatory variable (that is, the i-th variable) is judged not suitable when the error of the comparison is large. The i-th variable is adopted when the results CV is more optimized than the current best value $CY_0$ (YES in S20), and the best value $CV_0$ is updated by the result CV (S22). However, when the result CV obtained is larger than the best value $CV_0$ (NO in S20), the i-th variable is not adopted (S24). Then, the process returns to step S14 to repeat the same processing. This processing is repeated until no improvement is found on the cross validation (NO in S26). The result CV is discriminatively assessed stepwise by increasing (adding) or decreasing (excluding) the explanatory variable to be adopted one after another in the correlation model. In other words, each explanatory variable is selected by sequentially judging whether the explanatory variable to be added to the model improves fitting in total. This process is repeated until no improvement is observed in fitting in total. The flow returns to the start at step S14 with i=1 when the result is improved by the processing above, and the selection of the explanatory variables is repeated based on the explanatory variables already selected. The data are analyzed in the above-mentioned processing by using a data set or a training set as one of two data sets divided in advance from a data set in order to decide predictable power of the model. Since the analysis gives the above-mentioned result by using the training set, a test set is predicted from the result to assess fitting of the measured data (S28). Although such assessment is not always required, it is effective for deciding the predictable power.

Fig. 3 shows a flowchart of the calculation (Fig. 2, S18) of the cross validation CV including the leave-one-out processing. The result of cross validation of the selected variables is calculated in this process. At first, the initial value of PRESS is set to 0 (S180). Then, the number j indicating a sample in a set of n data is set to 1 (S182), and the partial least square method is applied to n-1 samples except the j-th sample (S184) to predict the object variable of the j-th sample (S186). A square of the difference is calculated and added to provide PRESS (S190). Subsequently, one is added to the number j (S182) and s similar operation is repeated. This processing is repeated for each sample until j = n. PRESS obtained in this way is a sum of squares of the difference between the predicted value calculated by excluding one sample sequentially and the measured value, and represents a predicted error. The value having an inverse sign of the predicted residual error sum of squares (PRESS) is defined to be the cross-validation result CV (S192).

The cross validation (CV = -PRESS) is optimized in this embodiment while the input variables (explanatory variables) are added or excluded stepwise one after another by using the cross-validation method. Five model construction methods are described below as examples for easy recognition of the stepwise addition and exclusion of the explanatory variables. They differ from each other in the procedures of sequential selection of the explanatory variables.

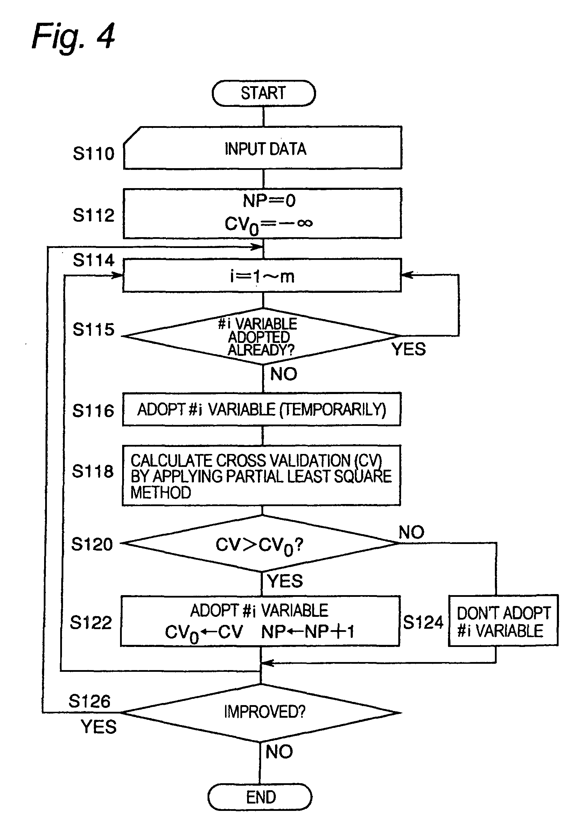

Fig. 4 shows a first method for constructing a model. An initial sate is defied when any explanatory variables are not selected in the data set (S112). Then, it is discriminated whether the cross validation is improved or not by selecting an explanatory variable (S120) while an assessment step of the cross validation (S 118) is repeated with the leave-one-out processing for every explanatory variables that have not been selected yet in the first to last (m-th) explanatory variables, and the explanatory variable is added when the cross validation is improved (S114 to S124). The sequential discrimination operation is repeated from the first explanatory variable until no improvement and addition are possible (NO in S126).

In more detail, the number NP of the selected explanatory variables is set to 0 while the best value $CV_0$ of the cross validation CV is set to $-\infty$ (S112) as the initial setting. The explanatory variables are then selected. The variable i is set to 1 (S114), and the i-th variable is temporarily adopted (S116). However, the process returns to step S114 when the i-th variable has been already adopted (YES in S115). Subsequently, the cross validation CV is calculated by applying the partial least square method (S118) using the leave-one-out processing. The i-th variable is adopted when the results CV obtained is more improved than the best value $CV_0$, and the former best value $CV_0$ is renewed by the result CV (S122). However, the i-th variable is not adopted (S124) when the results CV obtained is not larger than the best value $CV_0$ (NO in S120). Then, the process returns to step S114, and the same processing is repeated. This processing is repeated until the cross validation CV is not improved any more (NO in S126). When the result is improved by the above procedure, the process returns to step S 114 again, and starts a new loop. The selection of the variables is repeated herein based on the variables that have been selected before. A correlation model is obtained by using the variables selected by the data set.

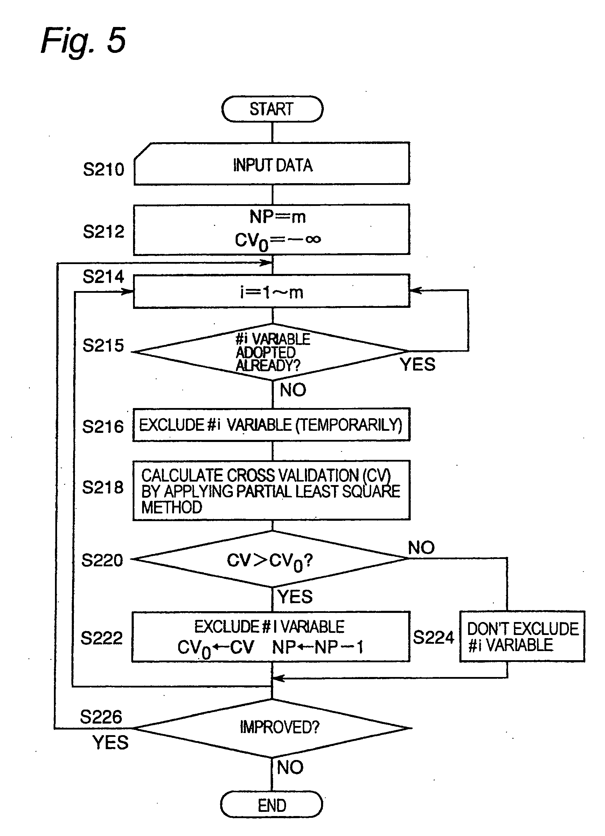

Fig. 5 shows a second method for constructing a model. The initial state is defined when all the explanatory variables are selected (S212). Then, whether the cross validation is improved or not by excluding an explanatory variable is discriminated (S220) while an assessment step (S218) of the cross validation is repeated using the leave-one-out processing for every explanatory variable that have been selected yet in the first to last (m-th) explanatory variables, and the corresponding explanatory variable is excluded when improved (S214 to S224). The discrimination procedure is sequentially repeated from the first explanatory variable until no improvement is observed (NO in S226).

In more detail, the number NP of the selected explanatory variables is set to m while the best value $CV_0$ of the cross validation CV is set to $-\infty$ (S212) as the initial setting. In other word, all explanatory variables are then selected. Then, the explanatory variables are selected. The variable i is set to 1 (S214), and the i-th variable is temporarily excluded (S216). However, the process returns to step S214 when the i-th variable has been already excluded (YES in S215). Subsequently, the cross validation CV are calculated by applying the partial least square method (S218). Then, the leave-one-out processing is applied. The i-th variable is excluded if the results CV obtained is more optimized than the best value $CV_0$ (YES in S220), and the former best value $CV_0$ is renewed by the result CV (S222). On the other hand, the i-th variable is not excluded (S224) when the results CV obtained is not larger than the best value $CV_0$ (NO in S220). Then, the flow returns to step S214, and the same processing is repeated. This processing is repeated until the cross validation CV is not improved any more (NO in S226). When the result is improved by the above procedure, the flow returns to step S214 again, and starts a new loop. Selection of the variables is repeated herein based on the variables that have been selected before. A correlation model is obtained by using the variables selected by the data set.

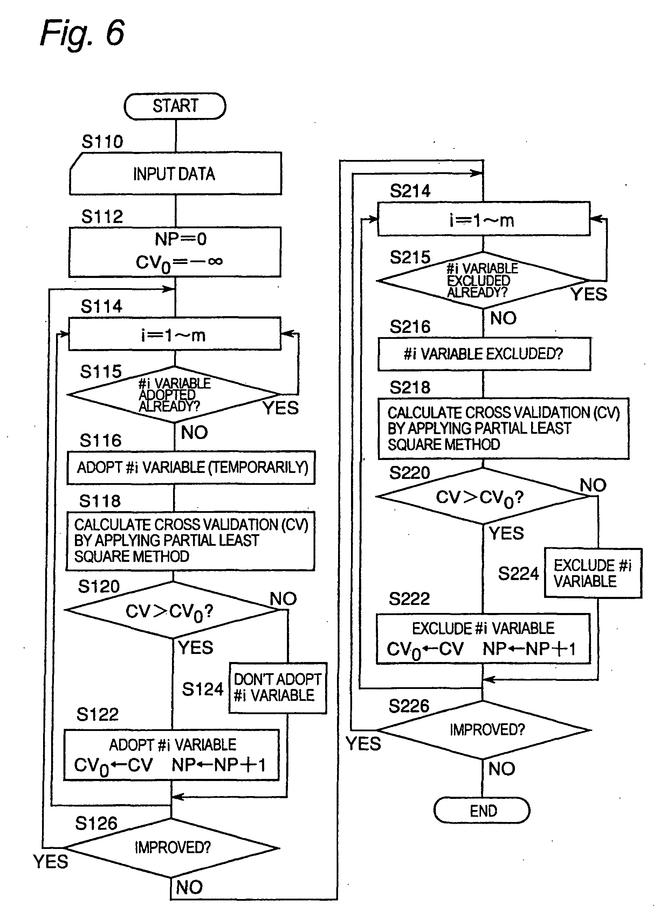

Fig. 6 shows a third method for constructing a model. This method is a combination of the first and second methods connected in series. The initial state is defined when no explanatory variables are selected (S112). Then, whether the cross validation is improved or not by selecting an explanatory variable is discriminated while assessment steps of the cross validation are sequentially repeated by using the leave-one-out processing for every non-selected explanatory variable of from the first explanatory variable to the last (m-th) one. The explanatory variable is additionally selected when the result is improved, and the sequential discrimination operations are repeated starting from the first explanatory variable until no improvement is observed by adding the variable (S114 to S126). Subsequently, whether the cross validation is improved or not by excluding an explanatory variable is discriminated while assessment steps of the cross validation are sequentially repeated using the leave-one-out processing for every selected explanatory variable of from the first explanatory variable to the last (m-th) one. The explanatory variable is excluded when the result is improved, and the sequential discrimination operations are repeated starting form the first explanatory variable until no improvements are observed by excluding the variable (S214 to S226).

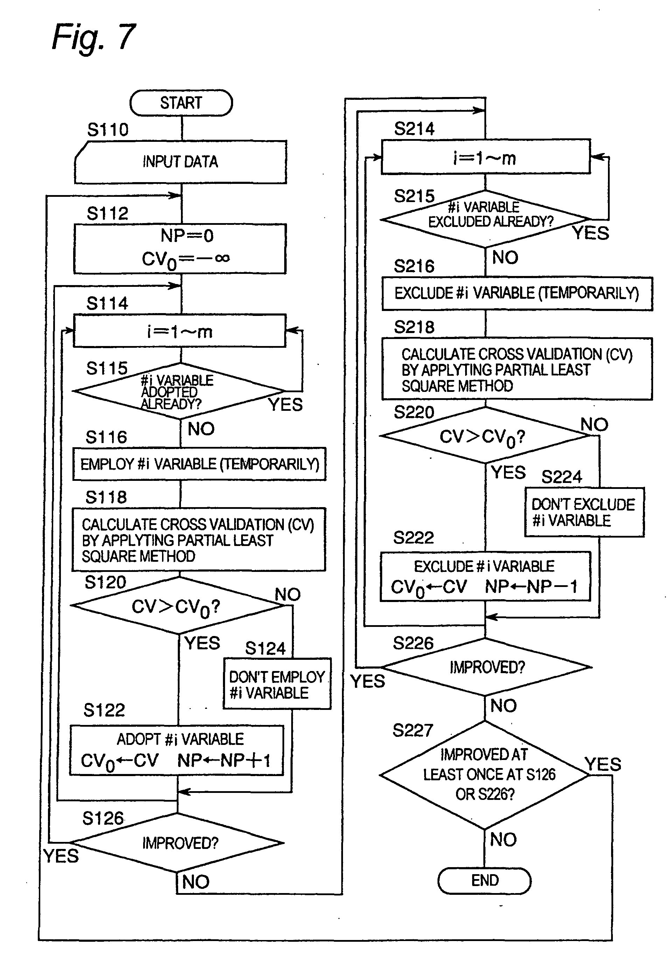

Fig. 7 shows a fourth method for constructing a model. This method is a modification of the third method. The initial state is defined when no explanatory variables are selected (S112). Then, it is discriminated whether the cross validation is improved or not by selecting an explanatory variable (S 120) while assessment step of the cross validation (S118) is sequentially repeated by using the leave-one-out processing for every non-selected explanatory variable of from the first explanatory variable to the last (m-th) one. The explanatory variable is additionally selected when the result is improved (S114 to S124), and the sequential discrimination operations are repeated starting form the first explanatory variable until no improvement is observed by adding the variable (NO in S126). Next, it is discriminated whether the cross validation is improved or not by excluding an explanatory variable (S220) while assessment step (S218) of the cross validation is sequentially repeated by using the leave-one-out processing for every selected explanatory variable of from the first explanatory variable to the last (m-th) one. The explanatory variable is excluded (S214 to S224) when the result is improved, and the sequential discrimination operations are repeated starting form the first explanatory variable until no improvement is observed by excluding the variable (NO in S226). The flow returns to step S112 when the result is improved at least once (YES in S227) in the sequentially discriminated addition improvement step or sequentially discriminated exclusion improvement step, and the above operations (S112 to S227) are repeated. This flow is repeated until no improvement is observed (NO in S227).

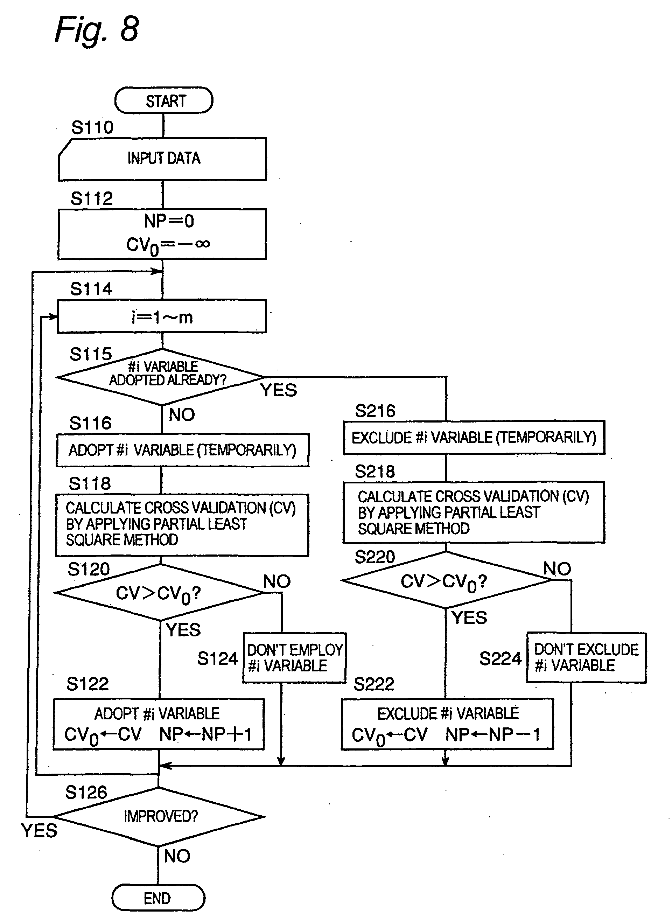

Fig. 8 shows a fifth method for constructing a model. This method is a parallel combination of the first and second schemes. The initial state is defined when no explanatory variables are selected (S112). Then, it is discriminated whether the cross validation is improved or not by selecting an explanatory variable (S120) while assessment step of the cross validation (S118) is sequentially repeated by using the leave-one-out processing for every non-selected explanatory variables of from the first explanatory variable to the last (m-th) explanatory variable. The explanatory variable is added when the result is improved (S114 to S124). On the other hand, it is discriminated whether the cross validation is improved or not by excluding an explanatory variable (S220) while assessment steps (S218) of the cross validation are sequentially repeated when the explanatory variable has been already selected. The explanatory variable is excluded (S216 to S224) when the result is improved, and the sequential discrimination operations are repeated starting form the first explanatory variable until no improvements are observed by adding or excluding the variable (NO in S 126).

The case when the fourth method for constructing the model (Fig. 7) is applied is described below by using the data set in Table 1 as an example. A correlation model corresponding to this data set is determined by analysis with the partial least square method. The number n of the data in Table 1 is 10, and the number m of the explanatory variables are reduced to only 19 for easy explanation. In Table 1, p1 represent the object variable, and p2 to p20 represent the explanatory variables (data in p16 and thereafter are omitted in Table 1 for the convenience of presentation). In contrast to steps S114 and S214 in the fourth method (Fig. 7), i representing an explanatory variable is sequentially processed in the inverse order of p20 to p2. The predicted residual error sum of squares (PRESS) is adopted herein for assessing the CV value. The smaller PRESS means better assessment of the CV value. The number NP of the explanatory variable is 0, and PRESS = $\infty$ (or $CV_0$ = -$\infty$) in the initial state.

Table 1:

| Data for 10 samples | | | | | | | | | | | | | | |
|---|---|---|---|---|---|---|---|---|---|---|---|---|---|---|
| # | p1 | p2 | p3 | p4 | p5 | p6 | p7 | p8 | p9 | p10 | p11 | p12 | p13 | p14 | p15 |
| 1 | 0.713 | 0.105 | 0.782 | 0.425 | 0.164 | 0.023 | 0.696 | 0.543 | 0.333 | 0.691 | 0.336 | 0.668 | 0.017 | 0.061 | 0.5 |
| 2 | 0.133 | 0.009 | 0.071 | 0.002 | 0.793 | 0.872 | 0.092 | 0.391 | 0.63 | 0.241 | 0.517 | 0.369 | 0.166 | 0.841 | 0.1 |
| 3 | 0.545 | 0.193 | 0.765 | 0.334 | 0.109 | 0.538 | 0.578 | 0.652 | 0.38 | 0.501 | 0.729 | 0.91 | 0.865 | 0.389 | 0.8 |
| 4 | 0.752 | 0.915 | 0.472 | 0.999 | 0.798 | 0.363 | 0.622 | 0.487 | 0.353 | 0.967 | 0.778 | 0.484 | 0.517 | 0.982 | 0.0 |
| 5 | 0.9 | 0.407 | 0.534 | 0.816 | 0.806 | 0.42 | 0.572 | 0.957 | 0.12 | 0.696 | 0.833 | 0.051 | 0.377 | 0.849 | 0.4 |
| 6 | 0.455 | 0.587 | 0.721 | 0.53 | 0.252 | 0.434 | 0.882 | 0.486 | 0.741 | 0.243 | 0.893 | 0.947 | 0.462 | 0.952 | 0.2 |
| 7 | 0.427 | 0.652 | 0.515 | 0.426 | 0.764 | 0.592 | 0.595 | 0.595 | 0.551 | 0.606 | 0.416 | 0.163 | 0.316 | 0.718 | 0.6 |
| 8 | 0.042 | 0.902 | 0.274 | 0.899 | 0.402 | 0.469 | 0.668 | 0.945 | 0.746 | 0.912 | 0.97 | 0.515 | 0.368 | 0.514 | 0.4 |
| 9 | 0.935 | 0.276 | 0.936 | 0.101 | 0.54 | 0.366 | 0.899 | 0.71 | 0.924 | 0.792 | 0.486 | 0.329 | 0.501 | 0.076 | 0.5 |
| 10 | 0.54 | 0.021 | 0.505 | 0.224 | 0.724 | 0.431 | 0.071 | 0.968 | 0.482 | 0.322 | 0.773 | 0.543 | 0.353 | 0.107 | 0.9 |

Table 2:

| Result of selection of variables in 10 steps on data in Table 1 | | | | | | | | | | |
|---|---|---|---|---|---|---|---|---|---|---|
| 0 | | | ∞ | - | | | | | | |
| 1 | ADD | p20 | 0.111 | p20 | | | | | | |
| 2 | ADD | p18 | 0.090 | p18 | & p20 | | | | | |
| 3 | ADD | p16 | 0.073 | p16 | & p18 | & p20 | | | | |
| 4 | ADD | p10 | 0.073 | p10 | & p16 | & p18 | & p20 | | | |
| 5 | ADD | p6 | 0.062 | p6 | & p10 | & p16 | & p18 | & p20 | | |
| 6 | ADD | p3 | 0.060 | p3 | & p6 | & p10 | & p16 | & p18 | & p20 | |
| 7 | ADD | p12 | 0.055 | p3 | & p6 | & p10 | & p12 | & p16 | & p18 | & p20 |
| 8 | EXCLUDE | p20 | 0.053 | p3 | & p6 | & p10 | & p12 | & p16 | & | |
| 9 | EXCLUDE | p10 | 0.050 | p3 | & p6 | & p12 | & p16 | & p18 | | |
| 10 | ADD | p13 | 0.048 | p3 | & p6 | & p12 | & p13 | & p16 | & p15 | |

The variables are treated in the inverse order of p20 to p2, as described above. The numbers in the leftmost column in Table 2 show 10 steps in which the result is improved by selecting the variables. "0" means the initial state. "Addition" and "Exclusion" in the next column mean operations in the addition loop and in the exclusion loop, respectively. The variable in the third column show the added or excluded variable. The figure in the fourth column are the result of the cross validation (division of PRESS by the number of the samples). The fifth column shows the variable (s) selected at the step.

There is no variable at all at the initial state, and PRESS is set to ∞ (infinity). As shown in Table 2, when p20 is adopted as explanatory variable first, PRESS is 0.111. Then the explanatory variable p20 is added since the result is improved over the initial value. Then, when p 19 is adopted as explanatory variable, p19 and p20 are used as the explanatory variables. However, p19 is not added since PRESS = 0.129 or the result is not improved. Next, p18 is added and PRESS = 0.090. Then the explanatory variable p18 is added, and p18 and p20 are used as explanatory variables. The procedure is continued thereafter similarly as shown in Table 2. (It is to be noted that p10 is added since the result is improved at fourth decimal place). When the first loop for the explanatory variables p20 to p2 is completed, the explanatory variables adopted include p3, p6, p10, p 16, p18 and p20, and the result is PRESS = 0.60. In the second loop, the explanatory variable p12 is added, and the result of PRESS = 0.55 is obtained. In the third loop, no improvement is observed by addition, and the additional processing of S114 to S126 are completed, and the flow proceeds to S214. The result of the partial least-square fitting and the prediction by leave-one-out processing at this moment are shown in Table 3.

Table 3 shows the result of the partial least-square fitting and leave-one-out prediction when the process is advanced to step 7 in Table 2. The error between the calculated value and measured value for model prediction and leave-one-out prediction are shown. A mean square of the errors, a square of the correlation coefficient R and a square of the predictable correlation factor Q are shown at the bottom of the table.

Table 3:

| Results of treatment at step 7 in Table 2 | | | | | |
|---|---|---|---|---|---|
| | Model prediction | | | Leave-one-out prediction | |
| # | Measured value | Calculated value | Error | Calculated value | Error |
| 1 | 0.713 | 0.757 | -0.044 | 0.693 | 0.020 |
| 2 | 0.133 | -0.056 | 0.189 | -0.051 | 0.184 |
| 3 | 0.545 | 0.497 | 0.048 | 0.480 | 0.065 |
| 4 | 0.752 | 0.646 | 0.106 | 0.495 | 0.257 |
| 5 | 0.900 | 0.687 | 0.214 | 0.557 | 0.343 |
| 6 | 0.455 | 0.489 | -0.034 | 0.512 | -0.057 |
| 7 | 0.427 | 0.624 | -0.198 | 0.672 | -0.245 |
| 8 | 0.042 | 0.349 | -0.307 | 0.517 | -0.475 |
| 9 | 0.935 | 0.865 | 0.070 | 0.782 | 0.153 |
| 10 | 0.154 | 0.197 | -0.044 | 0.285 | -0.132 |

Table 3: (continued)

| Results of treatment at step 7 in Table 2 | | | | | |
|---|---|---|---|---|---|
| | Model prediction | | | Leave-one-out prediction | |
| # | Measured value | Calculated value | Error | Calculated value | Error |
| | 0.093 | $R^2=0.744$ | 0.024 | $Q^2=0.407$ | 0.055 |

In the first loop starting from step S214 for exclusion of variables, the result is improved by excluding the variables p10 and p20. In the second loop, no improvement is observed, and the steps S214 to S226 are completed. Then, the flow returns to S112 again based on the decision at step S227. In the first loop in the next addition processing, only adding of p13 causes improvement, but in the second loop, but in the next exclusion processing no improvement is observed. The flow returns again to step S112, and the processing is completed by confirming that no improvement is observed in steps S114 to S126 and in steps S214 to S226. Thus, six variables of p3, p6, p12, p13, p16 and p18 are selected to obtain a result of PRESS = 0.048. The details are shown in Table 4.

Table 4 shows the partial least-square fitting and the leave-one-out prediction when the processing is advanced to step 10 in Table 2.

Table 4:

| Results of treatment at step 10 in Table 2 | | | | | |
|---|---|---|---|---|---|
| | Model prediction | | | Leave-one-out prediction | |
| # | Measured value | Calculated value | Error | Calculated value | Error |
| 1 | 0.713 | 0.771 | -0.058 | 0.663 | 0.050 |
| 2 | 0.133 | -0.013 | 0.146 | 0.041 | 0.092 |
| 3 | 0.545 | 0.610 | -0.065 | 0.595 | -0.050 |
| 4 | 0.752 | 0.524 | 0.228 | 0.380 | 0.372 |
| 5 | 0.900 | 0.696 | 0.205 | 0.543 | 0.357 |
| 6 | 0.455 | 0.591 | -0.137 | 0.623 | -0.168 |
| 7 | 0.427 | 0.638 | -0.211 | 0.696 | -0.269 |
| 8 | 0.042 | 0.189 | -0.147 | 0.268 | -0.226 |
| 9 | 0.935 | 0.841 | 0.094 | 0.756 | 0.179 |
| 10 | 0.154 | 0.209 | -0.055 | 0.294 | -0.140 |
| | 0.093 | $R^2=0.765$ | 0.022 | $Q^2=0.482$ | 0.048 |

The partial least square method is considered efficient when the number of the explanatory variables is large, but a result of PRESS = 0.124 is obtained as shown in Table 5 when the full variables of p20 to p2 are adopted as the explanatory variables. This means that the leave-one-out processing has a poorer result than the error from the average value (0.093).

Table 5:

| Results of treatment when full variables are adopted | | | | | |
|---|---|---|---|---|---|
| | Model prediction | | | Leave-one-out prediction | |
| # | Measured value | Calculated value | Error | Calculated value | Error |
| 1 | 0.713 | 0.712 | 0.001 | 0.527 | 0.186 |
| 2 | 0.133 | -0.073 | 0.206 | 0.222 | -0.090 |
| 3 | 0.545 | 0.561 | -0.016 | 0.538 | 0.007 |
| 4 | 0.752 | 0.656 | 0.096 | 0.351 | 0.402 |
| 5 | 0.900 | 0.691 | 0.209 | 0.432 | 0.469 |
| 6 | 0.455 | 0.519 | -0.064 | 0.562 | -0.107 |
| 7 | 0.427 | 0.583 | -0.156 | 0.629 | -0.203 |

Table 5: (continued)

| Results of treatment when full variables are adopted | | | | | |
|---|---|---|---|---|---|
| | Model prediction | | | Leave-one-out prediction | |
| # | Measured value | Calculated value | Error | Calculated value | Error |
| 8 | 0.042 | 0.430 | -0.388 | 0.724 | -0.682 |
| 9 | 0.935 | 0.794 | 0.140 | 0.480 | 0.454 |
| 10 | 0.154 | 0.182 | -0.029 | 0.457 | -0.303 |
| | 0.093 | | 0.029 | | 0.124 |
| | | $R^2$=0.684 | | $Q^2$=-0.330 | |

EXAMPLES

The invention is described in more detail hereinafter with reference to examples, but the invention is not restricted to the examples.

EXAMPLE 1: Data analysis of DLBCL patients by feature extraction considering cross validation of partial least square method

Data of 28 DLBCL (lymphoma) patients obtained from the home page (http://11mpp.nih.gov/lymphoma/) of P. O. Brown et al. are divided into a training set of data of 20 patients and a test set of data of 8 patients. The survival month is used as an object variable, and the values of log(ch1/ch2) of 12,832 spots out of 18,432 spots in 28 data in which both ch1 and ch2 are positive are used as explanatory variables.

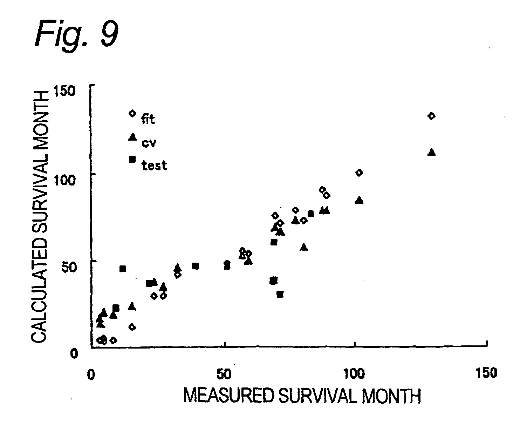

Determination of a partial least square model is attempted for the training set. Leave-one-out prediction ($Q^2$ > 0.5) in the partial least square method with the full 12,832 variables is not significant. Next, the explanatory variables are increased or decreased stepwise one after another so as to minimize the error of the leave-one-out prediction. The model construction method is similar to that in the third model except the order of addition and exclusion of the explanatory variables and the order of exclusion of the samples in the leave-one-out processing. No explanatory variable is selected in the initial state (S112). Then, a non-selected explanatory variable is selected sequentially among explanatory variables from the last (m-th) to the first variable, it is judged whether the result of cross validation with leave-one-out processing (wherein one sample from the last (n-th) to the first sample is excluded sequentially) is improved or not if the variable is selected in the model, and then the selected variable is added when the result is improved. The above procedure (S114 to S126) is repeated from the m-th explanatory variable until no improvement of the result of cross validation is observed by the addition of explanatory variable. Next, the explanatory variable in the model is selected among the variables from the last (m-th) to the first variable, it is judged whether the result of cross validation with leave-one-out processing (wherein one sample from the last (n-th) to the first sample is excluded sequentially) is improved or not if the variable is excluded from the model, and then the selected variable is excluded when the result is improved. The above procedure (S214 to S226) is repeated from the m-th explanatory variable until no improvement of the result of cross validation is observed by the exclusion of explanatory variable. As a result, a significant model ($R^2$ = 0.988, $Q^2$ = 0.895, NP = 342) is obtained. Fig. 9 shows the result of the partial least square method on the data, wherein rhombuses (fit) show the data of twenty patients in the training set, triangles (cv) show the data of the cross validation of the data. Further, squares show the data (8 patients) of the test set. The partial least square model obtained can predict 4/8 of the test set quite excellently, and 1/8 excellently.

In the above-mentioned multivariante analysis, the samples are the data obtained by using the DNA chip. However, the data analysis is not restricted to those obtained by using the DNA chip. It may readily be conjectured that the data analysis is also useful for data such as the gene expression level and the quantities of intracellular substances.

In Examples 2 to 7 explained below, usual statistical methods and multivariate analysis methods (such as a proportional hazard method, multiple regression analysis method, adaptive least square method, logistic regression method and linear discrimination analysis method) are applied to a relatively small number of explanatory variables selected by using the partial least square method.

Example 2: Survival time analysis of 240 DLBCL patients by feature extraction considering cross validation with partial least square method, and with proportional hazards analysis

DLBCL data set of 240 patients (Diffuse Large B-Cell Lymphoma) used is downloaded from the database open to the public on the web (http://llmpp.nih.gov/DLBCL/) by Rosenwald et al. All the data are used as the training set. Log $(\chi 1/\chi 2)$ are calculated with respect to 7,399 spots except those with $\chi 1$ or $\chi 2$ of 0 in the spot pattern and used as explanatory variables. In contrast to Example 1, the survival ratio ($P_{KM}$) at a time when an event happens is determined in this example by applying a life table according to the Kaplan-Meier method considering that a time terminating observation and death time are mixed in the survival time, and the object variable is defined to be a logic conversion of the survival ratio, $\log(P_{KM}/1 - P_{KM})$. While the life table according to the Kaplan-Meier method shows survival probability as a mass, a novel idea is used herein that the survival probability (probability for persons with no change survive) at the time of generation of the event in a mass including an individual j is recognized as a surviving time for the individual j at the time of generation of the event. This probability is used as an object variable by converting the probability into a logit value that express the tendency that a change happens. The leave-one-out method is used for the cross validation for the training set, and a partial least square model is obtained by sequentially selecting the parameters so as to decrease PRESS $\times 1.02^{NP}$. The partial least square model is obtained by improving the function PRESS $\times 1.02^{NP}$ in place of improving the cross validation (CV = -PRESS), as one of the functions having the cross validation as at least one of the independent variables. PRESS denotes a residual sum of squares of leave-one-out prediction, and NP denotes the number of the selected explanatory variables.

By selecting expressions of the following 19 genes as the explanatory variables, the processing is performed in which the cross validation CV in the flowchart in Fig. 7 are read as -PRESS $\times 1.02^{NP}$. Data ID as used herein shows the ID number in the original Web data. ACCESSION means an accession number in GenBank, and the line without the accession number corresponds to a gene (unknown) clarified only in the original data or EST, available by the method described in the paper.

| ACCESSION | data ID | comment |
|---|---|---|
| U03398 | #(27876) | tumor necrosis factor (ligand) superfamily, member 9 |
| M65066 | #(27394) | protein kinase, cMPA-dependent, regulatory, type I, beta |
| -- | #(27104) | (Unknown) |
| AK001546 | #(25048) | Homo sapiens cDNA FLJ10684 fis, clone NT2RP3000220 |
| -- | #(31372) | (Unknown) |
| U15085 | #(28178) | major histocompatibility complex, class II, DM beta |
| BC003563 | #(24983) | hypothetical protein MGC10796 |
| -- | #(16113) | (Unknown) |
| M23452 | #(16822) | small inducible cytokine A3 |
| | #(24433) | (Unknown) |
| X00437 | #(27480) | T cell receptor beta locus |
| U12979 | #(24377) | activated RNApolymerase II transcription cofactor 4 |
| X52479 | #(17773) | protein kinase C, alpha |
| H96306 | #(16578) | bone marrow stromal cell antigen 1 |
| U70426 | #(19255) | regulator of G-protein signaling 16 |
| AA830781 | #(33358) | EST |
| AA804793 | #(25022) | EST |
| H57330 | #(26383) | EST |
| S69790 | #(27184) | WAS protein family, member 3 |

A proportional hazards analysis is attempted with these gene expressions as candidates of the explanatory variables. It is a statistical method considering the time in the analysis of survival ratio. The analysis is carried out by using the program package JMP (JMP Sales SAS Campus Drive Carry, NC 27513 USA). The variables are narrowed further by a backward elimination method with $P \geq 0.05$ as a criterion for reducing the number of variables, and the following proportional hazard equation having expressions of 14 genes is obtained. Each term indicated by the accession number or data ID is the value of $\log(\chi 1/\chi 2)$ of each gene, and P is a hazard ratio for showing no statistical significance. The tendency of death is larger as the hazard value determined by the right side of this equation is larger.

$$hazard = 0.370 \text{ #(27104)} +0.589 \text{ AK001546} -0.366 \text{ #(31372)} -0.276 \text{ U15085}$$

$$-0.307 \text{ #(16113)} +0.409 \text{ M23452} -0.350 \text{ #(24433)} -0.297 \text{ X00437}$$

$$+0.321 \text{ U12979} -0.585 \text{ X52479} -0.457 \text{ U70426} +0.561 \text{ AA830781}$$

$$-0.430 \text{ H57330} +0.433 \text{ S69790}.$$

$P < 0.001$.

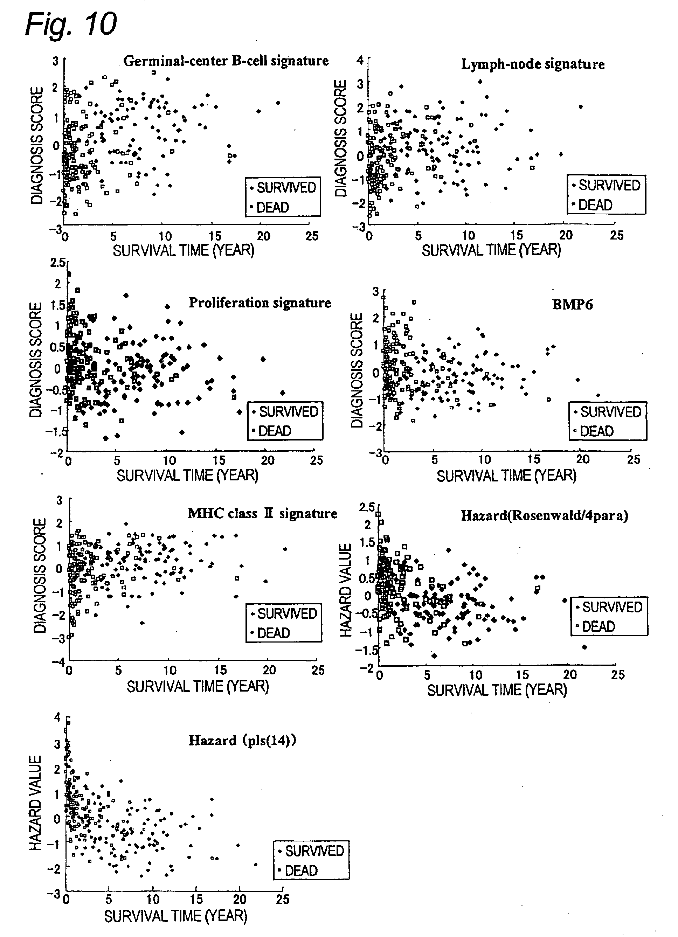

Rosenwald et al. selected diagnostic indices of five groups (seventeen genes) by using proportional hazards analysis of simple correlation. Fig. 10 shows comparison data for indicating to what extent the hazard values (indicated as Hazard (pls(14)) in Fig. 10) and the diagnostic indices obtained by Rosenwald et al. can explain the survival time. Since the proportional hazard equation with the parameters of the five groups by Rosenwald et al. involves a problem of statistical insignificance with the proliferation parameter $P > 0.05$, the hazard values of the parameters of four groups except an insignificant group are also shown for comparison (indicated as "Rosenwald/4 para" in the diagram). In Fig. 10, rhombuses show the data of dead or suspended patients, and squares show the data of surviving patients.

Correlation between the hazard value and survival time determined in this example is quite evident among these diagnostic indices. Since the hazard value decays with the survival time, a larger hazard value shows that the patient cannot survive longer. On the contrary, the indices obtained by Rosenwald et al. are found insufficient for judging the survival time. It is impossible to find an optimum parameter set from hundreds to thousands parameters by using only the proportional hazards analysis. However, an effective diagnostic index that could not be obtained before can be obtained by combining the feature extraction and the proportional hazed analysis by taking cross validation with the Kaplan-Meier method, logit transformation method and partial least square method into consideration. It is unexpected and of interest that such a good result is obtained by combining models having statistically different significance from one another. Prediction of survival time of patients brings about important information for deciding a therapeutic program and a life design including QOL, and a diagnosis model determined in this example is valuable in the society.

The following proportional hazard equation having six gene expressions is obtained by further narrowing the results by the backward elimination method with $P \geq 0.001$ as a criterion for excluding the variables. The number of the selected explanatory variables may be controlled by changing the criterion.

$$hazard = -0.426 \text{ U15085} +0.350 \text{ M23452} -0.521 \text{ X52479}$$

$$-0.450 \text{ U70426} -0.586 \text{ H57330} +0.476 \text{ S69790}.$$

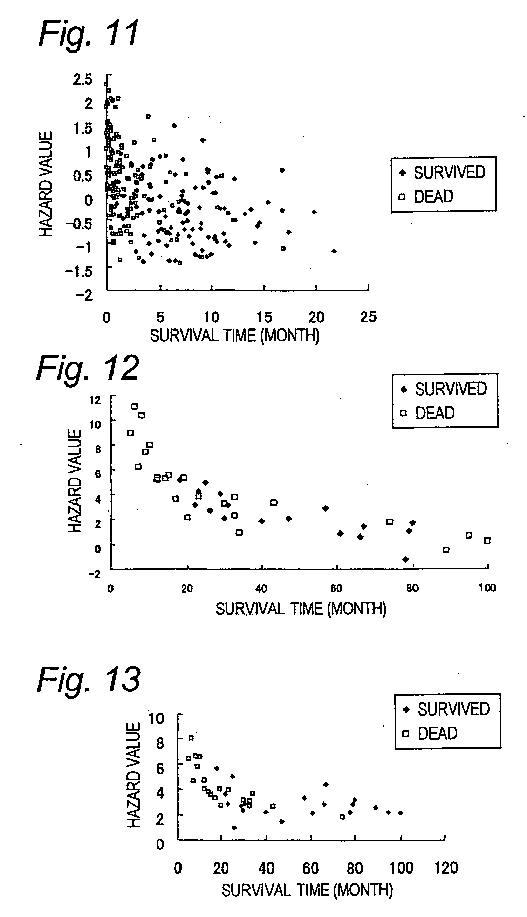

Fig. 11 shows a plot of the hazard value obtained by calculating the right side of the equation in the vertical axis against the survival time in the horizontal axis. In Fig. 11, the rhombuses show the data of dead or suspended patients, and the squares show the data of surviving patients, as in Fig. 10.

Example 3: Survival time analysis of forty breast cancer patients by feature extraction considering cross validation with partial least square method and proportional hazards analysis

The data set of breast cancer patients used is downloaded from a Web site (http://genome-www.stabford. edu./ breast_cancer/mopo_clinical/) of Sorlie et al. open to the public. All the data are used as a training set. While most of the data set comprises 40 and 24 patients measured with two kinds of DNA chips of type A and B, respectively, the data obtained with type A are used in this example. The logit value is determined from the survival time data as in Example 2 and used as object variables. LOG_RAT2N_MEAN values in 6,891 genes except genes with missing value of expression model are used as the explanatory variables. A partial least square model is obtained by sequentially selecting parameters so that the value of a function (PRESS $\times 1.13^{NP}$) of the cross validation and the number of the explanatory variables is decreased. This function is one of the functions having the cross validation as at least one of the independent variables. Expression of the following ten genes is selected as explanatory variables by reading the cross validation value CV in the flowchart in Fig. 7 as -PRESS $\times 1.13^{NP}$.

| ACCESSION | comment |
|---|---|
| AA406242 | (guanosine monophosphate reductase) |
| AA598572 | (spleen tyrosine kinase) |

(continued)

| ACCESSION | comment |
|---|---|
| H73335 | (Homo sapiens mRNA full length insert cDNA clone EUROIMAGE 980547 |
| W84753 | (Homo sapiens cDNA FLJ13510 fis, clone PLACE1005146) |
| AA703058 | (myeloperoxidase) |
| N71160 | (cytochrome c oxidase subunit Vib) |
| AA453345 | (a protein tyrosine kinase) |
| AA054669 | (Homo sapiens, clone IMAGE:3611719, mRNA, partial cds) |
| N32820 | (ESTs, weakly similar to ALU1_HUMAN ALU SUBFAMILY J SEQUENCE CONTAMINATION WARNING ENTRY [H. sapiens]) |
| R05667 | (suppressor of potassium transport defect 3) |

The backward elimination method with $P \geq 0.05$ as a variable reduction criterion in the proportional hazards analysis is attempted with the above-mentioned variables as candidates, and the following hazard equation having expressions of seven genes is obtained. Each term indicated by an accession number corresponds to LOG_RAT2N_MRAN of each gene.

$$hazard = -0.821 \text{ AA406242} +1.556 \text{ AA598572} -1.074 \text{ H7335} +1.418 \text{ W84753}$$

$$-1.290 \text{ AA703058} +2.182 \text{ N71160} +0.828 \text{ AA453345}.$$

$P < 0.001$. P of variables $< 0.05$.

Fig. 12 shows a plot of the hazard value obtained by calculating the right side of the equation in the vertical axis against the survival time in the horizontal axis. This also shows the hazard value to be a good diagnosis score. In Fig. 12 the rhombuses show the data of dead or suspended patients, and the squares show the data of surviving patients.

The variables are selected by the backward elimination method with $P \geq 0.001$ as a variable reduction criterion, and the following proportional hazard equation having expressions of three genes is obtained. The number of the explanatory variables can be controlled by changing the variable exclusion criterion.

$$hazard = 1.453 \text{ AA598572} -1.473 \text{ AA703058} +1.071 \text{ AA453345}.$$

Fig. 13 shows a plot of the hazard value obtained by calculating the right side of the equation in the vertical axis against the survival time in the horizontal axis. In Fig. 13, the rhombuses show the data of dead patients, and the squares show the data of surviving patients.

Example 4: Analysis to predict recurrence of forty breast cancer patients by using feature extraction considering cross validation with partial least square method and multiple regression analysis

The data of forty patients obtained by measuring expressions of 6,891 genes the DNA chip by Sorle et al. are used as a data set of the patient. Apartial least square model having eleven genes is obtained by sequentially selecting the parameters so that $PRESS \times 1.10^{NP}$ is reduced by using the incidence of recurrence as an object variable.

| ACCESSION | comment |
|---|---|
| AA434397 | integrin, beta 5 |
| T83209 | ESTs |
| N53427 | KIAA1628 protein |
| N29639 | cytidine monophosphate-N-acetylneuraminic acid hydroxylase |
| AA485739 | major histocompatibility complex, class II, |

(continued)

| ACCESSION | comment |
|-----------|---------|
|           | RD beta 5 |
| AA425861  | enoyl Coenzyme A hydratase 1, peroxisomal |
| H84871    | Ste-20 related kinase |
| T64312    | prostate cancer overexpressed gene 1 |
| T59518    | solute carrier family 2, (facilitated glucose transporter) member 8 |
| AA406231  | KIAA0381 protein |
| AA037488  | prolactin |

Then, discriminative analysis is performed by the multiple regression analysis method as one of the usual multivariate analysis methods wherein expressions of the selected genes are used as the explanatory variables and incidence of recurrence as the object variable. The analysis is executed by using the program package JMP. The following multiple regression analysis model is obtained by further selecting the variables by the backward elimination method with $P \geq 0.15$ as a criterion for reducing the variables. The significance level of recurrence is high when OLS calculated by this equation is positive, while it is low when negative.

$$OLS = -0.215 \text{ AA434397} +0.227 \text{ T83209} -0.209 \text{ N53427} +0.139 \text{ N29639}$$

$$+0.165 \text{ AA485739} +0.133 \text{ AA425861} -0.084 \text{ H84871} -0.193 \text{ T64312}$$

$$+0.237 \text{ T59518} +0.176 \text{ AA037488} -0.278.$$

$$R^2 = 0.84797, \text{ and rate of correct answer of discrimination } 97.5\%$$

Table 6 shows P values and decision coefficients when the above-mentioned discriminative analysis equation is prepared by using each one of the parameters.

Table 6

| Accession No. | P value | Decision coefficient ($R^2$) |
|---------------|---------|------------------------------|
| AA434397      | 0.0334  | 0.090273 |
| T83209        | 0.0601  | 0.066005 |
| N53427        | 0.0004  | 0.268678 |
| N29639        | 0.0552  | 0.069483 |
| AA485739      | 0.0421  | 0.080733 |
| AA425861      | 0.0861  | 0.05122 |
| H84871        | 0.087566 | 0.087566 |
| T64312        | 0.0004  | 0.263207 |
| T59518        | 0.0066  | 0.157196 |
| AA037488      | 0.0031  | 0.187627 |

There are three parameters not significant ($P > 0.05$) by themselves alone, and none of them has a small decision coefficient. Accordingly, the good discrimination equation as described above cannot be obtained merely by examining the parameters one after another. It is also impossible to efficiently find an optimum parameter set from hundreds to thousands parameters by using only the multiple linear repression analysis method. However, effective diagnostic indices can be obtained by feature extraction by taking the cross validation of the partial least square method into consideration. The prediction of recurrence of breast cancer is a problem desired in the society for planning and deciding a therapeutic program considering QOL.

Example 5: Analysis to predict recurrence of 40 + 24 breast cancer patients by feature extraction considering cross validation of partial least square method and adaptive least square method

Analysis is restricted to 3,448 genes common to type A (40 patients) and type B (24 patients) detected by the DNA chip. A partial least square model is obtained by sequentially selecting the parameters so as to reduce PRESS $\times 1.17^{NP}$. The following equation is obtained by discriminative analysis with the adaptive least square method by using expressions of the selected genes as explanatory variables. The significance level of recurrence is high when the ALS value calculated by the following equation is larger than 0.5.

$$\text{ALS} = 0.31\ H11482\ -0.29\ T64312\ -0.32\ AA045340\ +0.01.$$

$$R^2 = 0.65, eps = 0.13, and$$

rate of correct answer of discrimination = 90.0%.

H11482 is not significant in simple correlation as shown in Table 7, and it is a parameter that could be recognized only simultaneously with other parameters. Table 8 shows the result of prediction of type B patients by using the above-mentioned equation. Sensitivity and specificity of the discrimination equation are 81.8% and 53.8%, respectively, and statistically significant result of $\chi 2 = 3.233$ (5% < P < 10%) and ratio of correct answer of discrimination = 66.7%. These are encouraging results that recurrence of type B could be predicted with a significance level of not larger than 10% by using the model trained by the data of type A, although the data of type A and Type B seems to involve measurement errors ascribed to the different constructions of the DNA chips.

A partial least square model by using the following gene expressions as explanatory variables is obtained by selecting the parameters so as to reduce the value of PRESS $\times 1.12^{NP}$.

H11482, T64312, R99749, T65211, AA427625, AA455506.

The following discrimination equation is obtained by further selecting the variables as the candidates of the explanatory variables by using leave-one-out method as an index.

$$\text{ALS} = 0.53\ H11482\ -0.31\ T64312\ -0.33\ R99749\ -0.26\ AA455506\ +0.10.$$

$$R^2 = 1.00, eps = 0.10, and$$

rate of correct answer of discrimination = 100.0%.

A good discrimination equation as the above one could not be obtained only by examining the parameters one after another. It is impossible to efficiently find an optimum parameter set from hundreds to thousands parameters by using only any one of the adaptive least square method, logistic regression analysis and other discriminative analysis methods. However, an effective diagnostic index could be obtained by the feature extraction by taking the cross validation of the partial least square method into consideration.

Table 7:

| Interaction of parameters | | |
|---|---|---|
| Parameter | R | Nmis(/40) |
| H11482 | 0.361 | 14 |
| T64312 | 0.607 | 8 |
| AA045340 | 0.572 | 9 |
| T64312 & AA045340 | 0.716 | 6 |
| H11482 & T64312 & AA045340 | 0.804 | 4 |

Table 8:

| Prediction of 24 patients of type-B | | |
|---|---|---|
| Observed value | Predicted value | Frequency |
| - | - | 7 |
| + | - | 2 |
| - | + | 6 |
| + | + | 9 |

Example 6: Analysis to predict recurrence of 40 + 24 breast cancer patients by using feature extraction considering cross validation of partial least square method and logistic regression analysis or linear discriminant analysis

The following discrimination equation is obtained in the logistic regression analysis instead of the analysis with the first adaptive least square method used in Example 5.

LORA = 7.92 H11482 -5.69 T64312 -6.41 AA045340 -9.73.

$R^2$ = 0.63, $\chi^2$ = 35.00 (P < 0.0001), and rate of correct answer of

discrimination = 90.0%.

There would be a high risk of recurrence when the value of LORA calculated as the right side of the equation is positive. Though ratios of the coefficients and correlation coefficients are different from those in the adaptive least square method in Example 5, the results of discrimination of all the patients are the same. The prediction results of type B patients are also the same as those in Table 7.

The following discrimination equation is obtained by using the linear discriminant analysis instead of the adaptive least square methods used in Example 5.

LDA = 2.45 H11482-2.35 T64312 -2.56 AA045340 -4.03.

Rate of correct answer of discrimination = 80.0%

There would be a high risk of recurrence when the value of LDA calculated as the right side of the equation is positive. Though ratios of the coefficients and correlation coefficients are a little different from those in the adaptive least square method in Example 5, the results of prediction of all the patients are almost the same. The results of prediction of type B patients are also the same as those in Table 7.

It is used as the object variables in Examples 4, 5 and 6 whether the breast cancer recurs or not. Accordingly, the feature extraction method by taking the cross validation of the partial least square method into consideration is found effective when the object variable is the data for the nominal scale or ordinal scale. The nominal scale is used for classifying an object (sample) whether it belongs to a certain class or not, while the size and order among the classes are not considered. The ordinal scale is used for classifying an object into a specified class, while the classes are ordered as to the size, importance or the like.

Example 7: Analysis of recurrence time of forty breast cancer patients by using feature extraction considering cross validation of partial least square method and proportional hazards analysis

A partial least square model having expressions of nine genes is obtained by using logit values, similarly to the method used in Example 2, based on the time series data of recurrence, as object variables, similarly to the method used in Example 4 so as to reduce the value of PRESS $\times$ $1.15^{NP}$. Reduction of the number of the variables is attempted by using the measured values of gene expression as the explanatory variables in the proportional hazards analysis under a criterion of P $\geq$ 0.05, and the following proportional hazard equation having eight genes is obtained.

hazard = 1.122 AA448641 -1.781 R78516-1.434 R05934 +2.165 W84753

-1.923 AA629838 +2.665 H08581 +1.875 AA045730 +1.269 AI250654.

P < 0.0001.

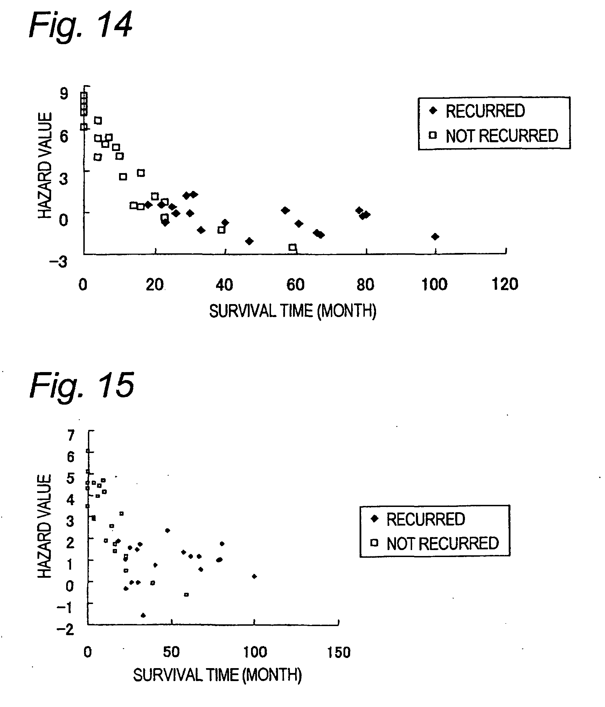

Fig. 14 shows a plot of the hazard value determined by calculating the right side of the equation in the vertical axis against the recurrence time in the horizontal axis. In Fig. 14, rhombuses denote the data of patients with no recurrence, and squares denote the data of patients with recurrence. Since the hazard value serves as an excellent diagnosis score, the method of the invention is found effective as a method for analyzing, not only survival time, but also change in a probabilistically generated biological condition with time.

By selecting the variables further by the variable reduction method with $\geq 0.005$ as a criterion for reducing the variables, the proportional hazard equation below having expressions of four genes is obtained.

$$\text{hazard} = 1.559 \text{ W84753} +2.265 \text{ H08581} +1.473 \text{ AA045730} +1.237$$

AI250654.

Fig. 15 shows a plot of the hazard value determined by calculating the right side of the equation in the vertical axis against the recurrence time in the horizontal axis. In Fig. 15, rhombuses denote the data of patients with no recurrence, and squares denote the data of patients with recurrence.

Example 8: Preparation of and measurement by a DNA chip for diagnosis of recurrence of breast cancer containing Genebank Accession numbers H11482, T64312 and AA045340

The DNA chip is prepared and used for measurements according to the method of Naohiko Seki, Tomomi Nagasugi, Takanori Azuma, Tsutomu Yoshikawa, Osamu Suzuki and Masaaki Muramatsu described in "Genomu kino kenkyu purotokoru", an extra issue of Jikken Igaku, Yodosha Co,. Ltd. (ISBN4-89705-932-7 C3047), p34-38. cDNAs with Genbank Accession numbers H11482, T64312 and AA045340 are used.

Each PCR product for a probe is precipitated with ethanol (Cat#057-00456, Wako Pure Chemical Industry, Co.) and is adjusted with DDW at concentration of 2 µg/µl. An equal volume of a DMSO solution of nitrocellulose (cat#41051-012, Gibco BRL) is added to the ethanol solution. After thorough mixing the PCR product is denatured by heating at 100°C for five minutes, it is quenched on ice. Then, the solution is warmed to room temperature and is spotted onto a sheet of carbodiimide slide glass (Nisshinbo Industries, Inc.) by using a DNA spotter SPBIO 2000 (Hitachi Software Engineering). After confirming that the spot is dried, the slide glass is subjected to ultra-violet cross-linking with a luminous energy of 60 mJ/cm$^2$ by using Ultraviolet Cross Linker (Amersham Pharmacia Biotech), and is stored at room temperature by vertically placing on a glass rack. The micro-array prepared above is immersed in a blocking solution having 3% BSA, 0.2 M NaCl, 0.1 M Tris (pH 7.5) and 0.005% Triton X-100, and it is left for about thirty minutes. Then, the solution adhered to the slide glass is drained well, and the slide glass is dried at 37°C. The slide glass is gently washed with a TE buffer (pH 8.0, cat#316-90025, Nippon Gene Co., Ltd.), and excess water is removed by gently centrifuging (1000 rpm, 1 minutes) the slide glass placed in a plate holder.

Next, mRNAs are purified from cell saps of normal mammary gland cell line SV-40 and breast cancer cell line MCF-7, MDA-MB-468 or T-47-D by using TRIZOL (cat#15596-018, Gibco BRL) and Oligotex dT30<super> (cat# W9021A, Takara Co.). Then, 2 µg of mRNA is dissolved in 6.4 µl of DEPC-treated DDW, and a solution having 9 µl of Oligo dT primer, 6 µl of 5x SuperScript II buffer (cat# 18089, Gibco BRL), 3 µl of DTT (within SuperScript), 0.6 µl of 50× dNTP, 3 µl of Cy3-dUTP (cat# PA53022, Amersham Pharmacia Biotech) or Cy5-dUTP (cat# PA55022, Amersham Pharmacia Biotech) and 2 µl of Superscript II is added to the mRNA solution. Then, it is reacted at 42°C for two hours. When one hour elapses after the reaction is started, 1 µl of Superscript II is added. 1.5 µl of an alkaline buffer (1N NaOH/20 nM EDTA) is added, and the reaction is continued at 65°C for 10 minutes, and 270 µl of TE buffer and 1.5 µl of 1N HCl are added to the solution. Then, both reaction solutions with Cy3 and Cy5 labels are mixed and transferred to a Microcon-YM-30 (cat#42410, Millipore/Amicon Co.). The sample is centrifuged at 10,000 rpm until the volume of the solution remaining in the upper cup is reduced to about 10 µl. The solution passing through the cup is transferred to another tube, and after adding 500 µl of TE buffer and 20 µg of Human Cot-1 DNA (cat# 15279-011, Gibco BRL) to the cup, the cup is centrifuged again until the volume is reduced to 10 µl or less. The cup is centrifuged at 3,000 rpm for three minutes to recover fluorescence-labeled DNA. The volume of the solution in the cup is adjusted to 20 µl by adding DDW, 50 µg of yeast RNA (cat# R7125, Sigma) and 40 µg of poly(A) (cat# 108 626, Roche Diagnostics), and it is transferred to a PCR tube. Further 4.25 µl of 20× SSC (cat# 15553-035, Gibco BRL) and 0.75 µl of 10% SDS (cat# 15553-035, Gibco BRL) are added to the tube, and the sample is denatured by heating at 100°C for one minute by using a PCR device. Then, the solution is slowly cooled by allowing the tube to stand at room temperature for thirty minutes.

The whole quantity of fluorescence-labeled DNA is placed on a cover glass, and it is put on the micro-array without mixing bubbles. The slide glass is placed in a hybridization chamber with a sheet of paper wiper wet with water and spread on the floor of the chamber. The chamber is hermetically sealed, and DNA is hybridized at 65°C overnight while gently shaking at two to four cycles per minute. The micro-array is taken out of the hybridization chamber, and it is gently dipped into 2× SSC/0.1% SDS solution in the state where the cover glass is placed on the micro-array. The micro-array is shaken for five minutes for waiting the cover glass to be spontaneously peeled. After the cover glass is peeled, the micro-array is placed in a slide glass rack, and it is washed again with 2× SSC/0.1% SDS solution by gently shaking for five minutes. The micro-array is further washed with 0.2× SSC/0.1% SDS twice at 40°C for five minutes, and it is rinsed with 0.2× SSC. The micro-array is transferred to a different sample case, and water on the microarray is removed by gently centrifuging it with a microtiterplate centrifuge (1000 rpm, 1 minute, room temperature). The micro-array is read with Scan Array 4000 (manufactured by GSI Luminonics Co.), and the data is analyzed by using an analysis software Qant Array (GSI Luminonics Co.) and Chip Space (Hitachi Software Engineering).

Example 9: Optimization of a partial least square model by genetic algorithm

The data on forty patients having measured expressions of 6,891 genes with DNA chip A used in Example 4 are used as a data set. The genetic algorithm is described, for example, in "Foundation of Genetic algorithm" by Tadashi Iniwa (Ohmsha, 1994). Explanatory variables are selected by using the genetic algorithm on the above data. The terms noted by " and " below are technical terms used usually in the field of genetic algorithm, and they are explained when necessary. The function PRESS × 1.01$^{NP}$ is used for "fitness". "Genotype" of each "individual" is presented as a sequence {b1, b2, b3, ...} which is 1 when an explanatory variable is used, and 0 when not used.

The size of a set of individuals is assumed to be 100. Initial "genotypes" (GTYPE) of individuals are prepared by using random numbers so that min_of (Ns, Ng, 300)/2 explanatory variables are adopted on the average, wherein Ns denotes the sample number (number of patients), Ng denotes the number of candidates of explanatory variables, and 300 is a constant for the convenience of practical execution.

Two individuals are selected from the set, and one of the individuals after "uniform crossover" of "genotype" is defined a new "individual". That is, any one (0 or 1) in the sequence of the "parent individuals" is selected with a probability of 1/2 for each "locus", and the selected sequence is substituted to create a new "individual". Subsequently, inversion of 0 ↔ 1 are carried out for every "loci" of the new "individual" with a probability of 1.1/(the number of adopted explanatory variables) when "0" is substituted (when the explanatory variable is adopted), and with a probability of 1.1/ ( the number of candidates of unadopted explanatory variables) when "1" is substituted (when explanatory variables are not adopted).

"Fitness" of the new "individual" prepared by the above-mentioned "crossover/mutation operation" is compared with "fitness" of an "individual" in the set that is a randomly selected as "opponent of tournament". The "individual" is substituted with a probability of 0.75 when the new "individual" is superior to the old one, and with a probability of 0.25 when the new "individual" is inferior to the old one. However, "elite strategy" is used here in which the substitution is prohibited when the "opponent of tournament" is the optimum solution in the set.

The set is optimized by repeating the cycle of "crossover" → "mutation" → "selection". The "number of generation" is defined herein as the division of the cycle number by the size of the set. The maximum "number of generation" is set to 100. The cycle is repeated by adding 10 to the "number of maximum generation" every time when a new optimum solution is found, until the "number of generation" reaches the maximum "number of generation".

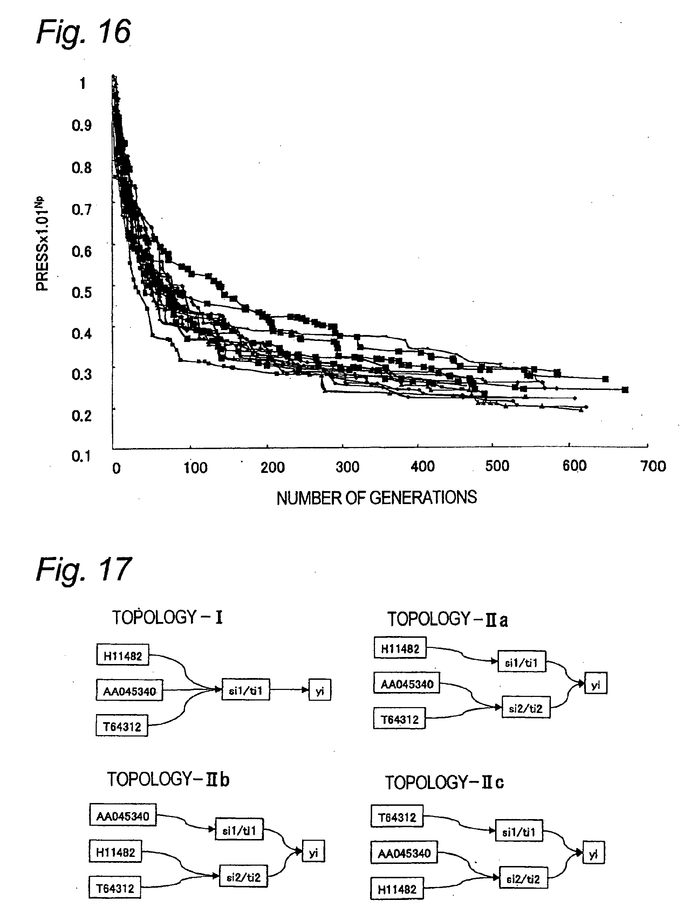

A series of the processing of the repetition of preparation of the initial set and optimization until the end is defined as a run, and 15 runs are performed. Fig. 16 summarizes how the optimization occurs in the 15 runs. The best result is obtained by using 25 explanatory variables.

Example 10: Construction of a model by hierarchical artificial neural network (MLP)

Three explanatory variables are obtained by the feature extraction with PLS-CV from 3,448 common genes to type Abreast cancer (40 patients) and type B breast cancer (24 patients) so that the function PRESS × 1.17$^{NP}$ is reduced, and they are used in the discriminative analysis of recurrence of the breast cancer patients in Example 5.

In the analysis method, the MLP has three layers with a structure wherein sigmoid conversion is performed only once in the intermediate layer (tk) , and four topologies in Fig. 17 are tried. Learning of weights in the network is performed by a back propagation algorithm.

$$s_{ik} = \sum_j w_{kj} \cdot P_{ij}$$

$$t_{ik} = 1/(1 + \exp(-s_{ik})).$$

$$y_i = \sum_j v_k \cdot t_{ik}.$$

The results of network topology I and IIb are as follows. Topology IIa and topology IIc are inferior to topology IIb.

Topology I:

$$y = 0.76 -1.77\ t1.$$

$$s1 = -12.48 - 42.89\ H11482\ +39.38\ AA045340\ +29.65\ T64312.$$

$$R^2 = 0.717. \qquad Q^2 = 0.142.$$

Topology IIb:

$$y = 1.19 - 0.86\ t1 - 1.43\ t2.$$

$$t1 = 2.65\ +18.25\ AA045340.$$

$$t2 = -0.40\ -2.29\ H11482\ +3.55\ T64312.$$

$$R^2 = 0.626. \qquad Q^2 = 0.416.$$

Example 11: Construction of a proportional hazards model with latent variables

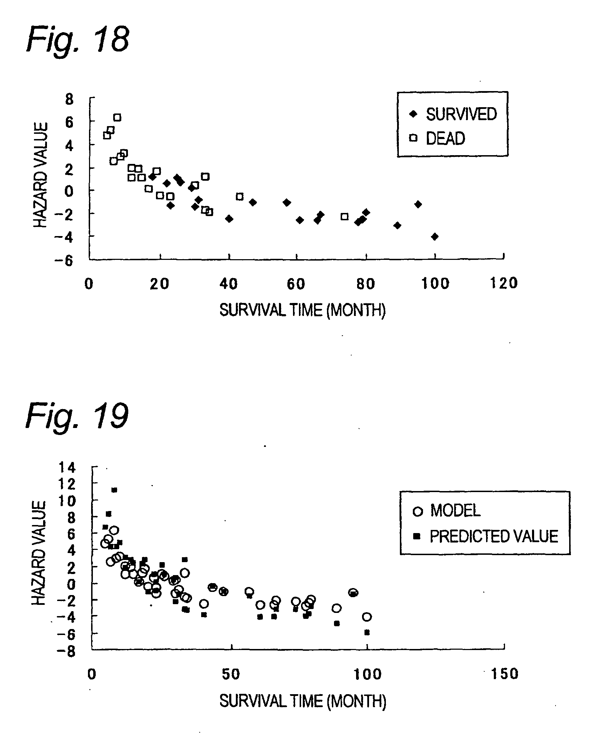

One latent variable produced in the analysis process of PLS is extracted by using the expression levels of ten genes selected in the PLS-CV method used in Example 3 as explanatory variables, and by using the logit value of the survival ratio as an object variable. When the analysis of the proportional hazard model us attempted with the latent variable as the explanatory variable, the equation obtained is significant with $P \leq 0.0001$. Fig. 18 shows a plot of the hazard value obtained by calculating the right side of the equation in the vertical axis against the survival time in the horizontal axis.

For assessing the prediction performance of the hazard equation obtained in this technique, the hazard equation is produced by using remaining 39 cases after excluding one case among the 40 cases used, and the hazard value of the excluded one case is predicted. Fig. 19 shows a plot between the values predicted by the hazard equation from 39 cases, and calculated values of the hazard equation from 40 cases. Fig. 19 shows that the technique can give a good result in the prediction of the hazard value.

Advantages of the invention are explained below. The number of variables can be decreased by selecting the explanatory variables with cross-validation when a correlation model is determined between the changes in the biological conditions probabilistically generated with time and the expression levels of genes and/or the quantities of intracellular substances. Thus, a good multivariate analysis model can be obtained with excellent prediction power. This method is particularly useful when the number of the explanatory variables is as large as 1,000 or more such as in the gene expression level. By decreasing the number of the variables, important genes and mechanisms operating in the background of diseases and biological phenomena can be predicted or specified and can be understood well. Inexpensive diagnostic materials such as DNA chips, DNA content vectors and antibody chips may be designed and provided by restricting only to important gene products and intracellular substances.

Further, a correlation model between the changes in the biological conditions probabilistically generated with time and the expression levels of genes and/or the quantities of intracellular substances can be determined by using the quantities derived from the changes in the biological conditions probabilistically generated with time.

**EP 1 498 825 A1**

Further, conventional statistical methods or multivariate analysis methods can be applied when the number of the explanatory variables is reduced by using the partial least square method.

## Claims

1. A data analysis system for determining a correlation model with a biological condition or a change in the biological condition probabilistically generated with time as an object variable and gene expression levels and/or quantities of intracellular substances as explanatory variables, the system comprising:

   an input means for receiving a sample set including data on the biological condition or data from which the biological condition is derived, or data on the change in the biological condition probabilistically generated with time, and the expression levels of a plurality of genes and/or the quantities of intracellular substances;

   (A) a selection means for selecting explanatory variables;
   (B) a calculation means for calculating a result of cross validation with executing a partial least square method, or for calculating a result of cross validation with executing a partial least square method by using a conversion result as the object variable, the conversion result being obtained by converting a probability that no change occurs by applying a life table according to Kaplan-Meier method or Cutler-Ederer method to the data on the change in the biological condition, based on or without an assumed distribution;
   (C) a judging means for judging adoption and non-adoption of the explanatory variables by assessing the result of the calculation by the calculation means; and
   (D) a decision means for deciding a partial least square model by continuously improving a function having the result of the cross validation of the partial least square model as at least one of independent variables by using the selection means (A), the calculation means (B) and the judging means (C).

2. The data analysis system according to Claim 1, wherein the object variable is the biological condition, the data received by said input means are the biological conditions or data from which the biological condition is derived, and the calculation means (B) calculates the cross validation with executing the partial least square method.

3. The data analysis system according to Claim 1, wherein the object variable is the change in the biological condition probabilistically generated with time, the data received by the input means are the data on the change in the biological condition probabilistically generated with time, and the calculation means (B) calculates calculating a result of cross validation with executing a partial least square method by using a conversion result as the object variable, the conversion result being obtained by converting a probability that no change occurs by applying a life table according to Kaplan-Meier method or Cutler-Ederer method to the data on the change in the biological condition, based on or without an assumed distribution;

4. The data analysis system according to Claim 1, 2 or 3, further comprising a final model decision means for constructing a model of a statistical method or multivariate analysis method by using the explanatory variables adopted in the partial least square model determined by the decision means or latent variables of the partial least square model.

5. The data analysis system according to any one of Claims 1 to 4, wherein the explanatory variables are sequentially adopted or excluded by the selection means.

6. The data analysis system according to any one of Claims 1 to 4, wherein the explanatory variables are selected by using a genetic algorithm by the selection means.

7. The data analysis system according to any one of Claims 1 to 6, wherein the result of the cross validation is calculated with the partial least square method by excluding a sample in the calculation means sequentially.

8. The data analysis system according to any one of Claims 1 to 6, wherein the cross validation is calculated by applying the partial least square method by excluding a plurality of samples in the calculation means sequentially.

9. The data analysis system according to Claim 7 or 8, wherein the calculation means determines a representative value of an error between the object variable showing the biological condition predicted by the gene expression of the excluded sample in each calculation and the object variable showing the biological condition of the excluded

26

sample, and uses the error as an index of the cross validation.

10. The data analysis system according to any one of Claims 1 to 9, wherein the function is the result of the cross validation.

11. The data analysis system according to any one of Claims 1 to 9, wherein the function of the result of the cross validation and the number of the selected explanatory variables is used.

12. The data analysis system according to Claim 5, wherein the decision means repeats discriminative assessment by improving a function having the result of the cross validation as at least one of the independent variables.

13. The data analysis system according to any one of Claims 1 to 12, wherein the selection means (A) and the calculation means (B) comprise a plurality of computers.

14. A data analysis system further comprising another input means for receiving the correlation model determined in Claims 1, 2, 3 or 4 and the explanatory variables adopted in the model on samples to be predicted, and a means for predicting and discriminating the biological condition of the samples based on the received explanatory variables.

15. The data analysis system according to Claim 2 wherein the object variable is the biological condition represented in nominal scale, ordinal scale or a continuous quantity.

16. The data analysis system according to Claim 4, wherein the statistical method or multivariate analysis method is a regression analysis method applied to a proportional hazard method or parametric distribution.

17. A data analysis method for determining a correlation model with a biological condition or a change in the biological condition probabilistically generated with time as an object variable and gene expression levels and/or quantities of intracellular substances as explanatory variables, the method comprising the steps of:

receiving a sample set including data on the biological condition or data from which the biological condition is derived, or data on the change in the biological condition probabilistically generated with time, and the expression levels of a plurality of genes and/or the quantities of intracellular substances;

(A) selecting the explanatory variables;
(B) calculating a result of cross validation by executing a partial least square method, or calculating a result of cross validation with executing a partial least square method by using a conversion result as the object variable, the conversion result being obtained by converting a probability that no change occurs when a life table is applied according to Kaplan-Meier method or Cutler-Ederer method to the data on the change in the biological condition, based on or without an assumed distribution;
(C) judging adoption and non-adoption of the explanatory variables by assessing the result of the calculation by the calculation in the calculating step (B); and
(D) deciding a partial least square model by continuously improving a function having the result of the cross validation of the partial least square model as at least one of independent variables by using the selecting step (A), the calculating step (B) and the judging step (C).

18. The data analysis method according to Claim 17, wherein the object variable is the biological condition, the data received in said input step are the biological conditions or data from which the biological condition is derived, and the cross validation is calculated in the calculation step (B) with executing the partial least square method.

19. The data analysis method according to Claim 17, wherein the object variable is the change in the biological condition probabilistically generated with time, the data received in the input step are the data on the change in the biological condition probabilistically generated with time, and in the calculating step (B) the result of cross validation is calculated with executing a partial least square method by using a conversion result as the object variable, the conversion result being obtained by converting a probability that no change occurs by applying a life table according to Kaplan-Meier method or Cutler-Ederer method to the data on the change in the biological condition, based on or without an assumed distribution.

20. The data analysis method according to Claim 17, 18 or 19 further comprising a step for constructing a model of a statistical method or a multivariate analysis method by using the explanatory variables adopted in the partial least square model determined by the decision means or latent variables of the partial least square model.

21. The data analysis method according to any one of Claims 17 to 20, wherein the explanatory variables are sequentially adopted or excluded in the selection step.

22. The data analysis method according to any one of Claims 17 to 20, wherein the explanatory variables are selected by using a genetic algorithm.

23. The data analysis method according to any one of Claims 17 to 22, wherein the result of the cross validation is calculated with the partial least square method by excluding one sample in the calculation step sequentially.

24. The data analysis method according to any one of Claims 17 to 22, wherein the result of the cross validation is calculated with the partial least square method by excluding a plurality of samples in the calculation step sequentially.

25. The data analysis method according to Claim 23 or 24, wherein in the calculation step a representative value of an error between the object variable showing the biological condition predicted by the gene expression of the excluded sample in each calculation and the object variable showing the biological condition of the excluded sample is determined, and the error is used as an index of the cross validation.

26. The data analysis method according to any one of Claims 17 to 25, wherein the function is the result of the cross validation.

27. The data analysis method according to any one of Claims 17 to 25, wherein the function of the result of the cross validation and the number of the selected explanatory variables is used.

28. The data analysis method according to Claim 21, wherein in the decision step discriminative assessment is repeated by improving the function having the result of the cross validation as at least one of independent variables.

29. The data analysis method according to any one of Claims 17 to 28, wherein the selection step (A) and the calculation step (B) are executed in a plurality of computers.

30. A data analysis method comprising the steps of

receiving the correlation model determined in Claims 17, 18, 19 or 20 and explanatory variables adopted in the model on samples to be predicted, and
predicting and discriminating the biological condition of the samples based on the received explanatory variables.

31. The data analysis method according to Claim 18 wherein the object variable is the biological condition represented in nominal scale, ordinal scale or a continuous quantity.

32. The data analysis method according to Claim 20, wherein the statistical method or multivariate analysis method used in the final model decision step is a regression analysis method applied to a proportional hazard method or parametric distribution.

33. A data analysis program executable in a computer for determining a correlation model with a biological condition or a change in the biological condition probabilistically generated with time as an object variable and gene expression levels and/or quantities of intracellular substances as explanatory variables, the program comprising the steps of

receiving a sample set including data on the biological condition or data from which the biological condition is derived, or data on the change in the biological condition probabilistically generated with time, and the expression levels of a plurality of genes and/or the quantities of intracellular substances;

(A) selecting the explanatory variables;
(B) calculating a result of cross validation by executing a partial least square method, or calculating a result of cross validation with executing a partial least square method by using a conversion result as the object variable, the conversion result being obtained by converting a probability that no change occurs by applying a life table according to Kaplan-Meier method or Cutler-Ederer method to the data on the change in the biological condition, based on or without an assumed distribution;

(C) judging adoption and non-adoption of the explanatory variables by assessing the result of the calculation by the calculation in the calculating step (B); and

(D) deciding a partial least square model by continuously improving a function having the result of the cross validation of the partial least square model as at least one of independent variables by using the selecting step (A), the calculating step (B) and the judging step (C).

34. The data analysis program according to Claim 33, wherein the object variable is the biological condition, the data received in said input step are the biological conditions or data from which the biological condition is derived, and the cross validation is calculated in the calculation step (B) with executing the partial least square method.

35. The data analysis program according to Claim 33, wherein the object variable is the change in the biological condition probabilistically generated with time, the data received in the input step are the data on the change in the biological condition probabilistically generated with time, and in the calculating step (B) the result of cross validation is calculated with executing a partial least square method by using a conversion result as the object variable, the conversion result being obtained by converting a probability that no change occurs by applying a life table according to Kaplan-Meier method or Cutler-Ederer method to the data on the change in the biological condition, based on or without an assumed distribution.

36. The data analysis program according to Claim 33, 34 or 35 further comprising a step for constructing a model of a statistical method or multivariate analysis method by using the explanatory variables adopted in the partial least square model determined by the decision means or latent variables of the partial least square model.

37. The data analysis program according to any one of Claims 33 to 36, wherein the explanatory variables are sequentially adopted or excluded in the selection step.

38. The data analysis program according to any one of Claims 33 to 36, wherein the explanatory variables are selected by using a genetic algorithm.

39. The data analysis program according to any one of Claims 33 to 38, wherein the result of the cross validation is calculated with the partial least square method by excluding a sample in the calculation step sequentially.

40. The data analysis program according to any one of Claims 33 to 38, wherein the cross validation is calculated by applying the partial least square method by excluding a plurality of samples in the calculation method sequentially.

41. The data analysis program according to Claim 39 or 40, wherein in the calculation step a representative value of an error between the object variable showing the biological condition predicted by the gene expression of the excluded sample in each calculation and the object variable showing the biological condition of the excluded sample is determined, and the error is used as an index of the cross validation.

42. The data analysis method according to any one of Claims 33 to 41, wherein the function is the result of the cross validation.

43. The data analysis method according to any one of Claims 33 to 41, wherein the function of the result of the cross validation and the number of the selected explanatory variables is used.

44. The data analysis program according to Claim 37, wherein in the decision step discriminative assessment is repeated by improving the function having the result of the cross validation as at least one of independent variables.

45. The data analysis program according to any one of Claims 33 to 44, wherein the selection step (B) and the calculation step (C) are executed in a plurality of computers.

46. A data analysis program comprising the steps of:

receiving the correlation model determined in Claims 33, 34, 35 or 36 and the explanatory variables adopted in the model on samples to be predicted, and

predicting and discriminating the biological condition of the samples based on the received explanatory variables.

**47.** The data analysis method according to Claim 34 wherein the object variable is the biological condition represented in nominal scale, ordinal scale or a continuous quantity.

**48.** The data analysis method according to Claim 36, wherein the statistical method or multivariate analysis method used in the final model decision step is a regression analysis method applied to a proportional hazard method or parametric distribution.

**49.** The program according to Claim 37, wherein the explanatory variables are not included at all in an initial state in the selection step.

**50.** The program according to Claim 37, wherein the full explanatory variables are included in the initial state in the selection step.

**51.** The program according to any one of Claims 37 to 50, wherein the biological condition comprise a measured value representing a type of disease, a measured value representing degree of critical of disease, a result of diagnosis representing a type of disease, a result of diagnosis representing degree of critical of disease, or a value derived from one of them.

**52.** A computer readable recording medium which records the program according to any one of Claims 33 to 48.

**53.** An intracellular substance measuring device, wherein the device detects expression of at least one gene selected substantially in a gene group of gene bank accession numbers U15085, M23452, X52479, U70426, H57330 and S69790, a detection method thereof, or a method for examining degree of critical of diffuse large cell lymphoma based on the detection.

**54.** The intracellular substance measuring device, the measuring method thereof, or the examining method according to Claim 53, wherein expression of at least one gene selected substantially from a gene group of gene bank accession numbers U03398, M65066, AK001546, BC003536, X00437, U12979, H96306, AA830781 and AA804793 is further detected.

**55.** An intracellular substance measuring device, wherein intracellular substances containing gene products comprising substantially genes of gene bank accession numbers AA598572, AA703058 and AA453345 are detected, a measuring method thereof, or a method for examining degree of critical of breast cancer based on the detection.

**56.** The intracellular substance measuring device, the measuring method thereof, or the examining method according to Claim 55, wherein intracellular substances containing substantially at least one gene product selected from a gene group of gene bank accession numbers AA406242, H73335, W84753, N71160, AA054669, N32820 and R05667 are further detected.

**57.** An intracellular substance measuring device wherein intracellular substances containing gene products comprising substantially genes of gene bank accession numbers W84753, H08581, AA045730 and AI250654 are detected, a measuring method thereof, and a method for examining recurrence of breast cancer based on the detection.

**58.** The intracellular substance measuring device, the measuring method thereof, or the examining method according to Claim 57, wherein the intracellular substances containing substantially at least one gene product of genes selected from a gene group consisting of gene bank accession numbers AA448641, R78516, R05934, AA629838 and H53037 are further detected.

**59.** An intracellular substance measuring device, wherein intracellular substances containing gene products substantially comprising genes of gene bank accession numbers AA434397, T83209, N53427, N29639, AA485739, AA425861, H84871, T64312, T59518 and AA037488 are detected, a measuring method thereof, and a method for examining recurrence of breast cancer based on the detection.

**60.** The intracellular substance measuring device, the measuring method thereof, or the examining method according to Claim 60, wherein intracellular substances containing gene products substantially of a gene of gene bank accession number AA406231 are detected further.

61. An intracellular substance measuring device, wherein intracellular substances containing gene products substantially of genes of gene bank accession numbers H11482, T64312 and AA045340, a measuring method thereof, and a method for examining recurrence of breast cancer based on the detection.

## Fig. 1

# Fig. 2

START

S10 — INPUT DATA

S12 — $NP = 0$ (AND OTHERS)
$CV_0 = -\infty$ (AND OTHERS)

S14 — $i = 1 \sim m$

S16 — ADOPT #i VARIABLE (TEMPORARILY)

S18 — CALCULATE CROSS VALIDATION (CV) BY APPLYING PARTIAL LEAST SQUARE METHOD

S20 — $CV > CV_0$?

NO

YES

S22 — ADOPT #i VARIABLE
$CV_0 \leftarrow CV$   $NP \leftarrow NP + 1$

S24 — DON'T ADOPT #i VARIABLE

S26 — IMPROVED?
YES

NO

S28 — PREDICT A TEST SET

END

*Fig. 3*

```
                    ( START )
                        |
   S180  |          PRESS=0          |
                        |
         |------------->|
   S182  |          j=1~n            |
         |              |
         |   | APPLY PARTIAL LEAST SQUARE |
   S184  |   | METHOD FOR N-1 SAMPLES    |
         |   | EXCEPT #j SAMPLE          |
         |              |
   S186  |   |   PREDICT #j SAMPLE       |
         |              |
         |   | MEASURE DEFFERENCE BETWEEN |
   S188  |   | VALUE OF #j SAMPLE AND     |
         |   | MEASURED VALUE             |
         |              |
   S190  |   |      ADD TO PRESS         |
         |              |
         |--------------|
                        |
   S192  |         CV=-PRESS          |
                        |
                    ( RETURN )
```

## Fig. 4

```
                      ┌──────────┐
                      │  START   │
                      └────┬─────┘
                           │
S110        ┌──────────────────────────────┐
            │         INPUT DATA            │
            └──────────────┬───────────────┘
                           │
S112        ┌──────────────────────────────┐
            │           NP=0                │
            │           CV_0=-∞            │
            └──────────────┬───────────────┘
                           │
S114        ┌──────────────────────────────┐
    ┌──────▶│            i=1~m             │◀──────┐
    │       └──────────────┬───────────────┘       │
    │                      │                        │
    │            ╱──────────────────╲               │
S115│           ╱   #i VARIABLE       ╲    YES       │
    │           ╲   ADOPTED            ╱────────────┘
    │            ╲  ALREADY?         ╱
    │             ╲────────┬───────╱
    │                   NO │
    │       ┌──────────────────────────────┐
S116│       │ ADOPT #i VARIABLE (TEMPORARILY)│
    │       └──────────────┬───────────────┘
    │                      │
    │       ┌──────────────────────────────┐
S118│       │ CALCULATE CROSS VALIDATION (CV)│
    │       │ BY APPLYING PARTIAL LEAST SQUARE│
    │       │ METHOD                         │
    │       └──────────────┬───────────────┘
    │                      │
    │            ╱──────────────────╲        NO
S120│           ╱    CV>CV_0?         ╲──────────┐
    │           ╲                    ╱            │
    │            ╲────────┬─────────╱             │
    │                 YES │                        │
    │       ┌──────────────────────────────┐  ┌─────────────┐
S122│       │       ADOPT #i VARIABLE       │  │ DON'T ADOPT │ S124
    │       │  CV_0←CV   NP←NP+1            │  │ #i VARIABLE │
    │       └──────────────┬───────────────┘  └──────┬──────┘
    │                      │◀──────────────────────────┘
    │            ╱──────────────────╲
S126│     YES   ╱    IMPROVED?        ╲
    └──────────╲                    ╱
                ╲────────┬─────────╱
                      NO │
                   ┌──────────┐
                   │   END    │
                   └──────────┘
```

# Fig. 5

```
                        ┌──────────┐
                        │  START   │
                        └────┬─────┘
                             │
S210 ──────────┌────────────────────────────┐
               │        INPUT DATA           │
               └──────────────┬──────────────┘
                              │
S212 ──────────┌──────────────────────────────┐
               │           NP=m                │
               │           CV₀=−∞              │
               └──────────────┬───────────────┘
                              │
S214 ──────────┌──────────────────────────────┐
               │           i=1~m               │
               └──────────────┬───────────────┘
                              │
                         ╱─────────╲
S215 ────────────────╱   #i VARIABLE  ╲    YES
                    ╲    ADOPTED      ╱──────────
                     ╲   ALREADY?    ╱
                       ╲───────────╱
                             │ NO
S216 ──────┌────────────────────────────────────┐
           │  EXCLUDE #i VARIABLE (TEMPORARILY)  │
           └─────────────────┬──────────────────┘
                             │
S218 ──────┌────────────────────────────────────┐
           │ CALCULATE CROSS VALIDATION (CV)     │
           │ BY APPLYING PARTIAL LEAST SQUARE    │
           │ METHOD                              │
           └─────────────────┬──────────────────┘
                             │
                        ╱─────────╲          NO
S220 ───────────────╱   CV>CV₀?    ╲────────────────
                     ╲            ╱
                       ╲────────╱
                          │ YES
S222 ──┌────────────────────────────────┐ S224 ┌──────────────┐
       │       EXCLUDE #i VARIABLE      │      │ DON'T EXCLUDE│
       │   CV₀←CV    NP←NP−1            │      │ #i VARIABLE  │
       └───────────────┬───────────────┘      └──────────────┘
                       │
                  ╱─────────╲
S226 ──────────╱  IMPROVED?  ╲
     YES        ╲           ╱
                  ╲───────╱
                     │ NO
                ┌─────────┐
                │   END   │
                └─────────┘
```

$NP=m$

$CV_0=-\infty$

$i=1\sim m$

$CV>CV_0?$

$CV_0 \leftarrow CV \quad NP \leftarrow NP-1$

EP 1 498 825 A1

## Fig. 6

**START**

S110 INPUT DATA

S112 $NP = 0$
$CV_0 = -\infty$

S114 $i = 1 \sim m$

S115 #i VARIABLE ADOPTED ALREADY? — YES

NO

S116 ADOPT #i VARIABLE (TEMPORARILY)

S118 CALCULATE CROSS VALIDATION (CV) BY APPLYING PARTIAL LEAST SQUARE METHOD

S120 $CV > CV_0$? — NO

YES

S124 DON'T ADOPT #i VARIABLE

S122 ADOPT #i VARIABLE
$CV_0 \leftarrow CV$   $NP \leftarrow NP + 1$

S126 IMPROVED?
YES — NO

S214 $i = 1 \sim m$

S215 #i VARIABLE EXCLUDED ALREADY? — YES

NO

S216 #i VARIABLE EXCLUDED?

S218 CALCULATE CROSS VALIDATION (CV) BY APPLYING PARTIAL LEAST SQUARE METHOD

S220 $CV > CV_0$? — NO

YES

S224 EXCLUDE #i VARIABLE

S222 EXCLUDE #i VARIABLE
$CV_0 \leftarrow CV$   $NP \leftarrow NP + 1$

S226 IMPROVED?
YES — NO

**END**

# Fig. 7

START

S110 INPUT DATA

S112
NP=0
$CV_0 = -\infty$

S114 $i = 1 \sim m$

S115 #i VARIABLE ADOPTED ALREADY? — YES

NO

S116 EMPLOY #i VARIABLE (TEMPORARILY)

S118 CALCULATE CROSS VALIDATION (CV) BY APPLYTING PARTIAL LEAST SQUARE METHOD

S120 $CV > CV_0$? — NO

YES

S124 DON'T EMPLOY #i VARIABLE

S122
ADOPT #i VARIABLE
$CV_0 \leftarrow CV$    $NP \leftarrow NP+1$

S126 IMPROVED?
YES
NO

S214 $i = 1 \sim m$

S215 #i VARIABLE EXCLUDED ALREADY? — YES

NO

S216 EXCLUDE #i VARIABLE (TEMPORARILY)

S218 CALCULATE CROSS VALIDATION (CV) BY APPLYTING PARTIAL LEAST SQUARE METHOD

S220 $CV > CV_0$? — NO

YES

S224 DON'T EXCLUDE #i VARIABLE

S222
EXCLUDE #i VARIABLE
$CV_0 \leftarrow CV$    $NP \leftarrow NP-1$

S226 IMPROVED?
YES
NO

S227 IMPROVED AT LEAST ONCE AT S126 OR S226? — YES

NO

END

## Fig. 8

```
                    ( START )
           ┌──────────┴──────────┐
  S110     │     INPUT DATA       │
           └──────────┬──────────┘
           ┌──────────┴──────────┐
  S112     │       NP=0           │
           │      CV₀=−∞          │
           └──────────┬──────────┘
           ┌──────────┴──────────┐
  S114     │      i=1~m           │
           └──────────┬──────────┘
                      ◇ S115
              #i VARIABLE
            ADOPTED ALREADY?  ──── YES ────┐
                      │ NO                 │
                      │                    │
  S116 ┌──────────────┴─────────┐   S216 ┌─────────────────────────────┐
       │ ADOPT #i VARIABLE       │        │ EXCLUDE #i VARIABLE          │
       │ (TEMPORARILY)           │        │ (TEMPORARILY)                │
       └──────────────┬─────────┘        └──────────────┬──────────────┘
  S118 ┌──────────────┴─────────┐   S218 ┌──────────────┴──────────────┐
       │ CALCULATE CROSS         │        │ CALCULATE CROSS VALIDATION  │
       │ VALIDATION (CV) BY       │        │ (CV) BY APPLYING PARTIAL    │
       │ APPLYING PARTIAL LEAST   │        │ LEAST SQUARE METHOD         │
       │ SQUARE METHOD            │        │                             │
       └──────────────┬─────────┘        └──────────────┬──────────────┘
  S120        ◇                    S220        ◇
          CV>CV₀?  ──── NO              CV>CV₀?  ──── NO
              │ YES      │                  │ YES     │
```

## Fig. 9

## Fig. 10

## Fig. 11

## Fig. 12

## Fig. 13

## Fig. 14

## Fig. 15

*Fig. 16*

*Fig. 17*

## Fig. 18

## Fig. 19

**EP 1 498 825 A1**

**INTERNATIONAL SEARCH REPORT**

| International application No. |
| --- |
| PCT/JP03/04059 |

A. CLASSIFICATION OF SUBJECT MATTER
Int.Cl⁷ G06F17/18, G06F17/30, C12N15/00, C12Q1/68, G01N33/574

According to International Patent Classification (IPC) or to both national classification and IPC

B. FIELDS SEARCHED

Minimum documentation searched (classification system followed by classification symbols)
Int.Cl⁷ G06F17/00-17/18, G06F17/30, C12N15/00-15/90,
C12Q1/00-1/70, G01N33/48-33/98

Documentation searched other than minimum documentation to the extent that such documents are included in the fields searched

| | | |
| --- | --- | --- |
| Jitsuyo Shinan Koho | 1922-1996 | Toroku Jitsuyo Shinan Koho | 1994-2003 |
| Kokai Jitsuyo Shinan Koho | 1971-2003 | Jitsuyo Shinan Toroku Koho | 1996-2003 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
| --- | --- | --- |
| A | WO 02/25405 A2 (THE REGENTS OF THE UNIVERSITY OF CALIFORNIA), 28 March, 2002 (28.03.02), Full text; all drawings (Family: none) | 1-52 |
| A | WO 00/70340 A2 (KAROLINSKA INNOVATIONS AB.), 23 November, 2000 (23.11.00), Full text; all drawings & EP 1179175 A3 | 1-52 |
| A | P. Gramatica, et al., QSAR STUDY ON THE TROPOSPHERIC DEGRADATION OF ORGANIC COMPOUNDS. In: Chemosphere 1999, Vol.38, No.6, pages 1371 to 1378 | 1-52 |

☒ Further documents are listed in the continuation of Box C. ☐ See patent family annex.

| | | | |
| --- | --- | --- | --- |
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier document but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| 27 June, 2003 (27.06.03) | 08 July, 2003 (08.07.03) |

| Name and mailing address of the ISA/ | Authorized officer |
| --- | --- |
| Japanese Patent Office | |
| Facsimile No. | Telephone No. |

Form PCT/ISA/210 (second sheet) (July 1998)

**INTERNATIONAL SEARCH REPORT**

| International application No. |
|---|
| PCT/JP03/04059 |

C (Continuation).   DOCUMENTS CONSIDERED TO BE RELEVANT

| Category* | Citation of document, with indication, where appropriate, of the relevant passages | Relevant to claim No. |
|---|---|---|
| A | J. Khan et al., Classification and diagnostic prediction of cancers using gene expression profiling and artificial neural networks. In: NATURE MEDICINE, 2001, Vol.7, No.6, pages 673 to 679 | 1-52 |
| P,A | A. ROSENWALD et al., THE USE OF MOLECULAR PROFILING TO PREDICT SURVIVAL AFTER CHEMOTHERAPY FOR DIFFUSE LARGE-B-CELL LYMPHOMA. In: The New England Journal of Medicine, 20 June, 2002 (20.06.02), Vol.346, No.25, pages 1937 to 1947 | 53-61 |

Form PCT/ISA/210 (continuation of second sheet) (July 1998)

## INTERNATIONAL SEARCH REPORT

| International application No. |
|---|
| PCT/JP03/04059 |

**Box I    Observations where certain claims were found unsearchable (Continuation of item 2 of first sheet)**

This international search report has not been established in respect of certain claims under Article 17(2)(a) for the following reasons:

1. ☐ Claims Nos.:

    because they relate to subject matter not required to be searched by this Authority, namely:

2. ☐ Claims Nos.:

    because they relate to parts of the international application that do not comply with the prescribed requirements to such an extent that no meaningful international search can be carried out, specifically:

3. ☐ Claims Nos.:

    because they are dependent claims and are not drafted in accordance with the second and third sentences of Rule 6.4(a).

**Box II    Observations where unity of invention is lacking (Continuation of item 3 of first sheet)**

This International Searching Authority found multiple inventions in this international application, as follows:

The claimed inventions in the present application consist of 6 groups of inventions, i.e., the inventions according to claims 1 to 52, the inventions according to claims 53 and 54, the inventions according to claims 55 and 56, the inventions according to claims 57 and 58, the inventions according to 59 and 60 and the invention according to claim 61.

As reported in, for example, International Publication WO 00/70340 pamphlet (2000), it had been well known by a skilled person in the art to detect an important gene based on the analysis results of determining a partial least squares method model or the like. Such being the case, there is no (continued to extra sheet)

1. ☒ As all required additional search fees were timely paid by the applicant, this international search report covers all searchable claims.

2. ☐ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**    ☐    The additional search fees were accompanied by the applicant's protest.

☒    No protest accompanied the payment of additional search fees.

Form PCT/ISA/210 (continuation of first sheet (1)) (July 1998)

**INTERNATIONAL SEARCH REPORT**

International application No.

PCT/JP03/04059

Continuation of Box No.II of continuation of first sheet(1)

technical relationship involving any "special technical feature" among these 6 groups of inventions and these groups of inventions are not considered as a group of inventions so linked as to form a single general inventive concept.

Form PCT/ISA/210 (extra sheet) (July 1998)